# EUROPÄISCHE PATENTSCHRIFT

(11) **EP 2 413 889 B1**
(45) Veröffentlichungstag und Bekanntmachung des Hinweises auf die Patenterteilung: **09.11.2016**
(21) Anmeldenummer: 10711394.6
(22) Anmeldetag: 29.03.2010
(51) Int. Cl.: A61K 8/37, A61Q 15/00, A61K 8/891

(54) **WASSERFREIE ANTITRANSPIRANT-SPRAYS MIT VERBESSERTER WIRKSTOFFFREISETZUNG**
WATER-FREE ANTIPERSPIRANT SPRAYS WITH IMPROVED ACTIVE SUBSTANCE RELEASE
SPRAYS ANTI-TRANSPIRANTS SANS EAU À LIBÉRATION AMÉLIORÉE DE SUBSTANCE ACTIVE

(30) Priorität: 01.04.2009 DE 102009002098
(43) Veröffentlichungstag der Anmeldung: 08.02.2012
(73) Patentinhaber: Henkel AG & Co. KGaA, 40589 Düsseldorf (DE)
(72) Erfinder: BANOWSKI, Bernhard, 40597 Düsseldorf (DE); BUSE, Nadine, 40724 Hilden (DE)
(86) Internationale Anmeldenummer: PCT/EP2010/054099
(87) Internationale Veröffentlichungsnummer: WO 2010/112460

(56) Entgegenhaltungen:
- EP-A1- 0 006 232
- EP-A1- 0 006 234
- FR-A1- 2 859 907
- US-A- 3 833 721

## Beschreibung

Gegenstand der vorliegenden Erfindung sind wasserfreie schweißhemmende Zusammensetzungen, insbesondere auf Basis einer wasserfreien, treibmittelfrei oder mit einem Treibmittel versprühbaren Suspension, die eine verbesserte Wirkstofffreisetzung des schweißhemmenden Wirkstoffs ermöglichen.

Wasserfreie, mit einem Treibmittel versprühbare Antitranspirant-Suspensionen enthalten neben schweißreduzierenden Wirkstoffen in der Regel mindestens ein kosmetisches Öl als Träger für den teilchenförmigen schweißreduzierenden Wirkstoff. Die Supensionen werden in einem druckfesten Behälter, meist einer Dose aus Weißblech oder Aluminium, die innen lackiert ist, zusammen mit einem verflüssigten Kohlenwasserstoff, wie n-Butan, iso-Butan und/oder Propan, als Treibmittel abgefüllt. Vor Gebrauch des Sprühventils, bei dem Treibmittel und eine Portion der Suspension freigesetzt werden, muss der Behälter zunächst ausreichend geschüttelt werden, um den abgesetzten Antitranspirantwirkstoff aufzumischen. Damit sich der suspendierte Antitranspirant-wirkstoff nicht sofort wieder absetzt, enthalten handelsübliche Suspensionen ein Suspendiermittel, z. B. hydrophob modifizierte Hectorite, wie sie beispielsweise unter den Handelsbezeichnungen Bentone Gel oder Bentone Powder von den Firmen Rheox und Elementis Specialties erhältlich sind.

Bei den handelsüblichen Sprays ist der in dem wasserfreien Träger suspendierte Antitranspirant-wirkstoff mit einer Ölschicht bedeckt. Während und nach der Applikation auf der Haut begünstigt diese Ölschicht das Sprühbild, das heißt, der Wirkstoff wird nicht zu sehr vernebelt, sondern gelangt gezielt auf die Haut; außerdem sorgt die Ölschicht für eine gewisse Haftung des pulverförmigen Antitranspirantwirkstoffs auf der Haut. Diese Ölschicht kann allerdings die Freisetzung des Antitranspirantwirkstoffs in die wirksame wasserlösliche Form verzögern. Insbesondere unpolarere Öle und/oder Öle mit einem niedrigen Löslichkeitsparameter tragen zu einer Verzögerung der Wirkstofffreisetzung bei.

Diesseitige Versuche, die unpolareren Öle und/oder Öle mit einem niedrigen Löslichkeitsparameter weitestgehend durch polare Öle und/oder Öle mit einem höheren Löslichkeitsparameter zu ersetzen, schlugen fehl: sofern die polare Öle und/oder Öle mit einem höheren Löslichkeitsparameter mindestens 50 Gew.-% des gesamten Trägeröls (Treibmittel ausgenommen), ausmachten, kam es zu einer ungleichmäßigen Entleerung des Produkts aus der Spraydose und auch das erzielte Sprühbild war für die bestimmungsgemäße Anwendung ungeeignet.

Cyclomethicone ist im Stand der Technik bereits als gut geeignetes Trägeröl für Antitranspirant-Sprays bekannt. Trotz seines niedrigen Löslichkeitsparameters erzielt man mit Cyclomethicone eine sehr zufrieden stellende Freisetzung des Antitranspirantwirkstoffs, da dieses Öl eine relativ hohe Flüchtigkeit aufweist und dadurch den Antitranspirantwirkstoff nicht zu stark blockiert. EP 6232A1 offenbart die Verwendung einer Kombination von a) adstringierenden sauren Aluminiumsalzen, b) Estern der Citronensäure, Acetylcitronensäure und/oder ein- oder zweibasischen Hydroxycarbonsäuren mit 2 bis 4 Kohlenstoffatomen mit aliphatischen oder alicyclischen C1-C6-Alkoholen und c) Antioxidantien in kosmetischen Mitteln zur Unterdrückung von Körpergeruch. Der Einsatz von Cyclomethicone sollte aufgrund seiner Persistenz möglichst vermieden werden. Eine Aufgabe der vorliegenden Anmeldung war es daher, unter weitestgehendem Verzicht auf Cyclomethicone versprühbare wasserfreie schweißhemmende Zusammensetzungen zu formulieren, die eine verbesserte Wirkstofffreisetzung des schweißhemmenden Wirkstoffs aufweisen.

Überraschend wurde nun gefunden, dass die Freisetzung des schweißhemmenden Wirkstoffs aus einer wasserfreien Antitranspirant-Zusammensetzung verbessert werden kann, wenn diese Triethylcitrat sowie mindestens ein weiteres unter Normalbedingungen flüssiges kosmetisches Öl als Träger enthält, wobei der Gewichtsanteil von Triethylcitrat an der Gesamtmenge an Ölen c) plus d) plus e), bezogen auf die gesamte treibmittelfreie Zusammensetzung, 40 - 50 Gew.-% beträgt und 0 bis weniger als 1 Gew.-% Cyclomethicone enthalten ist.

Gegenstand der vorliegenden Erfindung sind daher schweißhemmende Zusammensetzungen zur persönlichen Körperpflege, konfektioniert als treibmittelfrei versprühbare oder mit einem Treibmittel versprühbare Suspension, enthaltend mindestens einen schweißhemmenden Wirkstoff, wobei die schweißhemmenden Wirkstoffe in ungelöster, suspendierter Form vorliegen, 0 - 5 Gew.-% freies Wasser, bezogen auf das Gewicht der treibmittelfreien Zusammensetzung, weiterhin Triethylcitrat und mindestens ein weiteres unter Normalbedingungen flüssiges kosmetisches Öl als Träger, wobei der Gewichtsanteil von Triethylcitrat an der Gesamtmenge an Ölen 40 - 50 Gew.-% beträgt, weiterhin 0 - 3 Gew.-% Ethanol, bezogen auf das Gewicht der treibmittelfreien Zusammensetzung und 0 bis weniger als 1 Gew.-% Cyclomethicone, bezogen auf das Gewicht der treibmittelfreien Zusammensetzung, enthalten sind ist.

Die erfindungsgemäßen schweißhemmenden Zusammensetzungen auf wasserfreier Basis sind als treibmittelfrei versprühbare oder mit einem Treibmittel versprühbare Suspension konfektioniert.

Die erfindungsgemäße schweißhemmende Wirkstoffkombination eignet sich für versprühbar konfektionierte Zusammensetzungen, insbesondere für treibmittelhaltige Suspensionen, die als Antitranspirant-Spray angewendet werden.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Triethylcitrat ist in den erfindungsgemäßen Zusammensetzungen in einer solchen Menge enthalten, dass sein Gewichtsanteil am Gesamtölgehalt 40 - 50 Gew.-% beträgt.

Unter Gesamtölgehalt ist das Gewicht der Komponenten c) plus d) plus e) gemäß Anspruch 1 zu verstehen. Das Treibmittel ist nicht zu berücksichtigen.

Bezogen auf das Gewicht der gesamten treibmittelfreien Zusammensetzung, ist Triethylcitrat bevorzugt in einer Menge von 12 - 35 Gew.-%, besonders bevorzugt 19 - 30 Gew.-%, außerordentlich bevorzugt 22 - 25 Gew.-%, enthalten, wobei insbesondere auch ein Gehalt von 13, 14, 15, 16, 17, 18, 20, 21, 23 und 24 Gew.-% Triethylcitrat, bezogen auf das Gewicht der gesamten treibmittelfreien Zusammensetzung, bevorzugt sein kann.

Die erfindungsgemäßen Zusammensetzungen enthalten neben dem Triethylcitrat mindestens ein weiteres kosmetisches Öl, wobei der Gewichtsanteil des mindestens einen, von Triethylcitrat verschiedenen, Öls an der Gesamtmenge an Ölen 50 - 60 Gew.-% beträgt.

Duft- und Riechstoffe zählen erfindungsgemäß nicht zu den kosmetischen Ölen, die bei der Berechnung des Gewichtsanteils von Triethylcitrat am Gesamtölgehalt berücksichtigt werden. Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus- , Jasmin-, Patchouly-, Rosen-, Ylang-Ylang-Öl, Muskateller-, Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl und Sandelholzöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanumöl, Palmarosaöl, Patschuliöl, Perubalsamöl, Petitgrainöl, Pfefferöl, Pfefferminzöl, Piment-öl, Pine-Öl, Rosenöl, Rosmarinöl, Sandelholzöl, Sellerieöl, Spiköl, Sternanisöl, Terpentinöl, Thujaöl, Thymianöl, Verbenaöl, Wacholderbeeröl, Wermutöl, Wintergrünöl, Ysop-Öl, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Iso-eugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanillin, Veratrumaldehyd, Zimtaldehyd, Zimtalkohol, Zimtsäure, Zimtsäureethylester und Zimtsäurebenzylester.

Weitere (leichter flüchtige) Riechstoffe sind Alkylisothiocyanate (Alkylsenföle), Butandion, Limonen, Linalool, Linalylacetat und -propionat, Menthol, Menthon, Methyl-n-heptenon, Phellandren, Phenylacetaldehyd, Terpinylacetat, Zitral und Zitronellal.

Die Gesamtmenge an Triethylcitrat und mindestens einem weiteren, unter Normalbedingungen flüssigen kosmetischen Öl beträgt in erfindungsgemäß bevorzugten Antitranspirant-Zusammensetzungen 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte treibmittelfreie Zusammensetzung. Auch eine Gesamtmenge an Triethylcitrat und mindestens einem weiteren, unter Normalbedingungen flüssigen kosmetischen Öl von 56, 57, 58, 59, 60, 63, 65, 68, 70, 73, 75, 78 oder 80 Gew.-%, jeweils bezogen auf die gesamte treibmittelfreie Zusammensetzung, kann erfindungsgemäß besonders bevorzugt sein, wobei eine Gesamtmenge von 53 - 63 Gew.-%, bezogen auf die gesamte treibmittelfreie Zusammensetzung, besonders bevorzugt ist.

Bei den kosmetischen Ölen unterscheidet man flüchtige und nicht-flüchtige Öle. Unter nichtflüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 8000 Pa, außerordentlich bevorzugt 300 - 3000 Pa, aufweisen.

Die erfindungsgemäßen Zusammensetzungen sind unter anderem dadurch gekennzeichnet, dass sie 0 bis weniger als 1 Gew.-% Cyclomethicone, bezogen auf das Gewicht der treibmittelfreien Zusammensetzung, enthalten.

Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen einen Dampfdruck bei 20°C von ca. 13 - 15 Pa auf.

In einer bevorzugten Ausführungsform sind die erfindungsgemäßen Zusammensetzungen frei von flüchtigen linearen Siliconölen, insbesondere frei von flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning^{®} 200 (0,65 cSt) und Dow Corning^{®} 200 (1,5 cSt) von Dow Corning enthalten sind, und insbesondere frei von niedermolekularem Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Weitere bevorzugte erfindungsgemäße Zusammensetzungen sind dadurch gekennzeichnet, dass neben Triethylcitrat wegen des trockeneren Hautgefühls und der schnelleren Wirkstofffreisetzung mindestens ein flüchtiges Nichtsiliconöl enthalten ist. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen. Dieses mindestens eine C₈-C₁₆-Isoparaffin ist bevorzugt in einer Gesamtmenge von 25 - 50 Gew.-%, bevorzugt 30 - 45 Gew.-%, besonders bevorzugt 32 - 40 Gew.-%, außerordentlich bevorzugt 33, 34, 35, 36, 37, 38 oder 39 Gew.-%, jeweils bezogen auf die gesamte treibmittelfreie Zusammensetzung, enthalten.

Erfindungsgemäß bevorzugte Zusammensetzungen sind dadurch gekennzeichnet, dass das mindestens eine unter Normalbedingungen flüssige Trägeröl mindestens ein flüchtiges C₈-C₁₆-Isoparaffin, insbesondere Isononan, Isodecan, Isoundecan, Isododecan, Isotridecan, Isotetradecan, Isopentadecan und Isohexadecan sowie Mischungen hiervon, umfasst.

Weitere erfindungsgemäß bevorzugte Zusammensetzungen enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine.

Weitere erfindungsgemäß bevorzugte Zusammensetzungen enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine.

Weitere erfindungsgemäß bevorzugte Zusammensetzungen enthalten Triethylcitrat und eine Mischung aus Isodecan, Isoundecan, Isododecan und Isotridecan.

Neben Triethylcitrat und dem mindestens einen vorgenannten C₈-C₁₆-Isoparaffin enthalten weitere erfindungsgemäß bevorzugte Antitranspirant-Zusammensetzungen mindestens ein nichtflüchtiges kosmetisches Öl, ausgewählt aus nichtflüchtigen Siliconölen und nichtflüchtigen Nichtsiliconölen.

Das mindestens eine nichtflüchtige Öl gleicht den negativen Effekt des flüchtigen Isoparaffins auf das Rückstandsverhalten erfindungsgemäß bevorzugter Antitranspirant-Zusammensetzungen aus.

Durch die relativ schnelle Verdunstung der flüchtigen Öle können feste, unlösliche Bestandteile, insbesondere die Antitranspirantwirkstoffe, auf der Haut als unschöner Rückstand sichtbar werden.

Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden.

Selbstverständlich ist es ebenfalls möglich, erfindungsgemäße Antitranspirant-Zusammensetzungen mit einem geringen Anteil an flüchtigen Ölen - das heißt, mit 0,5 - 24,5 Gew.-% an flüchtigen Ölen, bezogen auf das Gewicht der treibmittelfreien Zusammensetzung - oder sogar ohne flüchtige Öle zu formulieren.

Als Begleitöl zu Triethylcitrat sind die nachfolgend genannten nichtflüchtigen Nichtsiliconöl-Typen besonders bevorzugt.

Erfindungsgemäß besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, Hexyldecylstearat (z. B. Eutanol^{®} G 16 S), Hexyldecyllaurat, Isononylisononanoat, 2-Ethylhexylpalmitat (z. B. Cegesoft^{®} C 24) und 2-Ethylhexylstearat (z. B. Cetiol^{®} 868). Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, Ethyl-hexylbenzoat, z. B. erhältlich als Finsolv^{®} EB, und Benzoesäureoctyldocecylester, z. B. erhältlich als Finsolv^{®} BOD.

Entsprechend bevorzugte Ölmischungen sind Triethylcitrat/2-Ethylhexylpalmitat, Triethylcitrat/ Hexyldecyllaurat, Triethylcitrat/2-Ethylhexylstearat, Triethylcitrat/Isopropylmyristat, Triethylcitrat/ Isopropylpalmitat, Triethylcitrat/2-Ethylhexyllaurat, Triethylcitrat/C₁₂-C₁₅-Alkyllactat, Triethylcitrat/ C₁₂-C₁₅-Alkylbenzoat und Triethylcitrat/Di-C₁₂-C₁₃-Alkylmalat. Besonders bevorzugte Ölmischungen sind Triethylcitrat/Isopropylmyristat, Triethylcitrat/Isopropylpalmitat und Triethylcitrat/C₁₂-C₁₅-Alkylbenzoat.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24.

Weitere bevorzugte nichtflüchtige Nichtsiliconöle sind ausgewählt aus Mischungen aus Guerbetalkoholen und Guerbetalkoholestern, z.B. dem Handelsprodukt Cetiol^{®} PGL (Hexyldecanol und Hexyldecyllaurat).

Weitere erfindungsgemäß geeignete nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Dicaprylylcarbonat (Cetiol^{®} CC). Ester der Kohlensäure mit C₁-C₅-Alkoholen, z. B. Glycerincarbonat oder Propylencarbonat, sind hingegen keine als kosmetisches Öl d) geeigneten Verbindungen. Propylencarbonat kann in den erfindungsgemäßen Zusammensetzungen gleichwohl enthalten sein, vornehmlich als Aktivator für das lipophile Verdickungsmittel. Sein Anteil ist allerdings nicht bei der Berechnung des Gewichtsanteils von Triethylcitrat an der Gesamtmenge an Ölen c) + d) + e) zu berücksichtigen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß bevorzugt sein, neben Triethylcitrat Mischungen der vorgenannten Öle einzusetzen. Weiterhin ist es besonders bevorzugt, Mischungen aus Triethylcitrat, mindestens einem flüchtigen C₈-C₁₆-Isoparaffin und mindestens einem Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, zu verwenden. In derartigen Mischungen sind die Ethylester und die Isopropylester besonders bevorzugt; außerordentlich bevorzugt sind Isopropylpalmitat und Isopropylmyristat. Weiterhin ist es besonders bevorzugt, Mischungen aus Triethylcitrat, mindestens einem flüchtigen C₈-C₁₆-Isoparaffin und mindestens einem Benzoesäureester von linearen oder verzweigten C₈₋₂₂-Alkanolen zu verwenden.

Erfindungsgemäß besonders bevorzugte Ölmischungen sind Triethylcitrat/2-Ethylhexylpalmitat/ Isodecan/Isoundecan/Isododecan/Isotridecan, Triethylcitrat/Hexyldecyllaurat/Isodecan/Isoundecan/Isododecan/Isotridecan, Triethylcitrat/2-Ethylhexylstearat/Isodecan/Isoundecan/Isododecan/ Isotridecan, Triethylcitrat/Isopropylmyristat/Isodecan/Isoundecan/Isododecan/Isotridecan, Triethylcitrat/Isopropylpalmitat/Isononan/Isodecan/Isoundecan/Isododecan/Isotridecan, Triethylcitrat/ 2-Ethylhexyllaurat/Isodecan/Isoundecan/Isododecan/Isotridecan, Triethylcitrat/C₁₂-C₁₅-Alkyllactat/ Isodecan/Isoundecan/Isododecan/Isotridecan, Triethylcitrat/C₁₂-C₁₅-Alkylbenzoat/Isodecan/Isoundecan/Isododecan/Isotridecan und Triethylcitrat/Di-C₁₂-C₁₃-Alkylmalat/Isodecan/Isoundecan/Isododecan/Isotridecan.

Weiterhin wurde gefunden, dass die Freisetzung des schweißhemmenden Wirkstoffs aus einer erfindungsgemäßen Antitranspirant-Zusammensetzung noch weiter verbessert werden kann, wenn mindestens ein Organosiloxan-Oxyalkylen-Copolymer enthalten ist.

Erfindungsgemäß bevorzugte Zusammensetzungen sind dadurch gekennzeichnet, dass das Organosiloxan-Oxyalkylen-Copolymer ausgewählt ist aus Verbindungen der allgemeinen Strukturformeln (I), (II), (III), (IV) und (V), 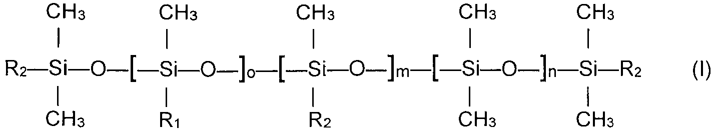 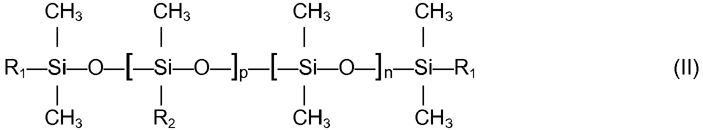 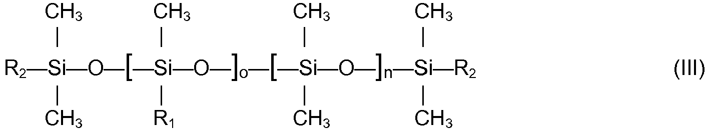 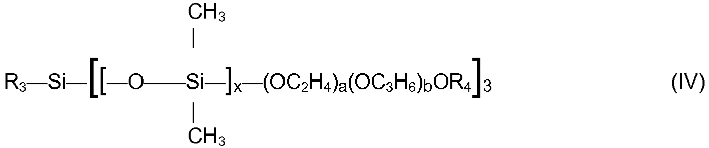 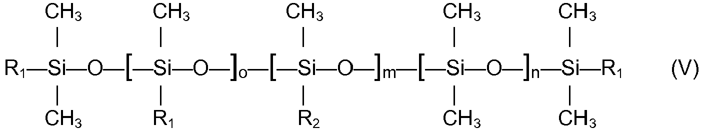 wobei
die Reste R¹ unabhängig voneinander eine lineare oder verzweigte C₁-C₃₀-Alkylgruppe oder eine ggf. substituierte Phenylgruppe, bevorzugt eine Methylgruppe, darstellen,
die Reste R² unabhängig voneinander die Gruppen -C_{c}H_{2c}-O-(C₂H₄O-)ₐ(C₃H₆O-)_{b}R⁵ oder -C_{c}H_{2c}-O-(C₂H₄O-)ₐR⁵ oder -CH₂-CH(CH₃)-CH₂-O-(C₂H₄O-)ₐ(C₃H₆O-)_{b}R⁵ darstellen,
die Reste R³ und R⁴ unabhängig voneinander eine lineare oder verzweigte C₁-C₁₆-Alkylgruppe und bevorzugt Methylgruppen darstellen,
die Reste R⁵ ein Wasserstoffatom oder eine Methylgruppe darstellen,
m eine Zahl von 0 - 20 darstellt,
n eine Zahl von 0 - 500, bevorzugt 20 - 400, besonders bevorzugt 50 - 300, darstellt,
o eine Zahl von 0 - 20 darstellt,
p eine Zahl von 1 - 50, bevorzugt 10 - 40, besonders bevorzugt 20 - 30, darstellt,
a eine Zahl von 0 - 50, bevorzugt 5 - 25, besonders bevorzugt 7 - 22, darstellt
b eine Zahl von 0 - 50, bevorzugt entweder 0 oder 5 - 30, besonders bevorzugt entweder 0 oder
10 - 25, außerordentlich bevorzugt entweder 0 oder 24, darstellt,
a + b mindestens 1 sind,
c eine Zahl von 1 - 4, besonders bevorzugt 3, darstellt,
x eine Zahl von 1 - 100 darstellt.

Bevorzugt für die erfindungsgemäße Lehre (Zusammensetzung, Verwendung, Verfahren) sind solche Organosiloxan-Oxyalkylen-Copolymere der oben dargestellten allgemeinen Strukturformeln (I), (II), (III) und (V), in denen die Reste R¹ unabhängig voneinander eine lineare oder verzweigte C₁-C₃₀-Alkylgruppe, bevorzugt eine lineare oder verzweigte C₁-C₁₆-Alkylgruppe, besonders bevorzugt eine lineare oder verzweigte C₁-C₄-Alkylgruppe, außerordentlich bevorzugt eine Methylgruppe, darstellen. Besonders bevorzugte lineare oder verzweigte C₁-C₄-Alkylgruppen sind ausgewählt aus Methyl, Ethyl, 1-Methylethyl, n-Propyl, n-Butyl, tert.-Butyl und 2-Methylpropyl.

Weiterhin bevorzugt für die erfindungsgemäße Lehre (Zusammensetzung, Verwendung, Verfahren) sind solche Organosiloxan-Oxyalkylen-Copolymere der oben dargestellten allgemeinen Strukturformel (IV), in denen die Reste R³ und R⁴ unabhängig voneinander eine lineare oder verzweigte C₁-C₁₆-Alkylgruppe, bevorzugt eine lineare oder verzweigte C₁-C₆-Alkylgruppe, besonders bevorzugt eine lineare oder verzweigte C₁-C₄-Alkylgruppe, außerordentlich bevorzugt eine Methylgruppe, darstellen. Besonders bevorzugte lineare oder verzweigte C₁-C₄-Alkylgruppen sind ausgewählt aus Methyl, Ethyl, 1-Methylethyl, n-Propyl, n-Butyl, tert.-Butyl und 2-Methylpropyl.

Weitere bevorzugte erfindungsgemäße Zusammensetzungen sind dadurch gekennzeichnet, dass das Organosiloxan-Oxyalkylen-Copolymer der oben dargestellten allgemeinen Strukturformeln (I), (II), (III), (IV) und (V) einen HLB-Wert im Bereich von 8 - 20, bevorzugt 10 - 18, besonders bevorzugt 11 - 16, aufweist.

Weitere bevorzugte erfindungsgemäße Zusammensetzungen sind dadurch gekennzeichnet, dass das Organosiloxan-Oxyalkylen-Copolymer der oben dargestellten allgemeinen Strukturformeln (I), (II), (III) und (V), in denen die Reste R¹ unabhängig voneinander eine lineare oder verzweigte C₁-C₃₀-Alkylgruppe, bevorzugt eine lineare oder verzweigte C₁-C₁₆-Alkylgruppe, besonders bevorzugt eine lineare oder verzweigte C₁-C₄-Alkylgruppe, insbesondere Methyl, Ethyl, 1-Methylethyl, n-Propyl, n-Butyl, tert.-Butyl und 2-Methylpropyl, außerordentlich bevorzugt eine Methylgruppe, darstellen und einen HLB-Wert im Bereich von 8 - 20, bevorzugt 10 - 18, besonders bevorzugt 11 - 16, aufweisen.

Weitere bevorzugte erfindungsgemäße Zusammensetzungen sind dadurch gekennzeichnet, dass das Organosiloxan-Oxyalkylen-Copolymer der oben dargestellten allgemeinen Strukturformel (IV), in denen die Reste R³ und R⁴ unabhängig voneinander eine lineare oder verzweigte C₁-C₁₆-Alkyl-gruppe, bevorzugt eine lineare oder verzweigte C₁-C₆-Alkylgruppe, besonders bevorzugt eine lineare oder verzweigte C₁-C₄-Alkylgruppe, insbesondere Methyl, Ethyl, 1-Methylethyl, n-Propyl, n-Butyl, tert.-Butyl und 2-Methylpropyl, außerordentlich bevorzugt eine Methylgruppe, darstellen und einen HLB-Wert im Bereich von 8 - 20, bevorzugt 10 - 18, besonders bevorzugt 11 - 16, aufweisen.

Erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methylgruppe, R² = -C_{c}H_{2c}-O-(C₂H₄O-)ₐR⁵,
R⁵ = ein Wasserstoffatom oder eine Methylgruppe,
n = 0, p = 1, a = 5 - 20, bevorzugt 7 - 15, besonders bevorzugt 8 - 11, b = 0, c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methylgruppe, R² = -C_{c}H_{2c}-O-(C₂H₄O-)ₐR⁵, R⁵ = ein Wasserstoffatom,
n = 0, p = 1, b = 0, a = 7, 8, 9, 10, 11, 12, 13, 14 oder 15, c = 3,
bevorzugt ausgewählt aus

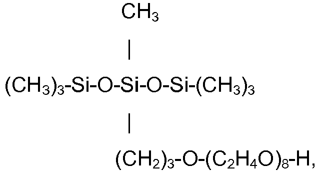

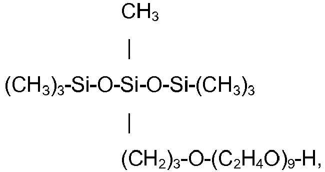

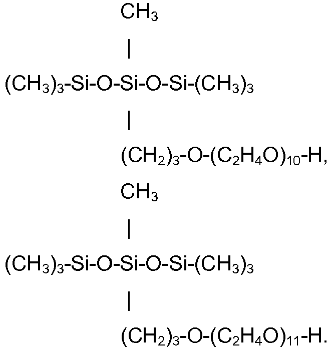

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = tert.-Butyl-Gruppe, R² = -C_{c}H_{2c}-O-(C₂H₄O-)ₐR⁵,
R⁵ = ein Wasserstoffatom oder eine Methylgruppe,
n = 0, p = 1, a = 5 - 20, bevorzugt 7 - 15, besonders bevorzugt 8 - 11, b = 0, c = 3,
bevorzugt ausgewählt aus

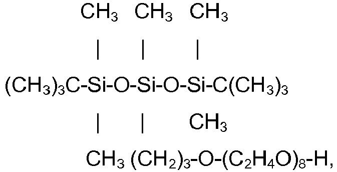

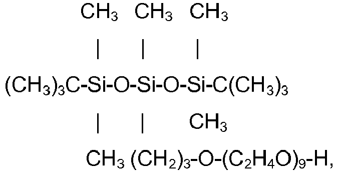

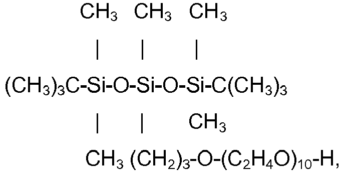

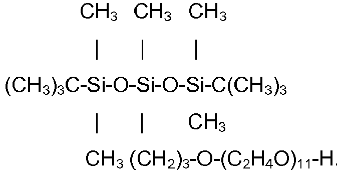

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = tert.-Butyl-Gruppen, R² = -C_{c}H_{2c}-O-(C₂H₄O-)ₐR⁵, R⁵ = ein Wasserstoffatom,
n = 0, p = 1, a = 7, 8, 9, 10, 11, 12, 13, 14 oder 15, c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Isopropyl-Gruppen (-CH(CH₃)₂), R² = -C_{c}H_{2c}-O-(C₂H₄O-)ₐR⁵,
R⁵ = ein Wasserstoffatom oder eine Methylgruppe,
n = 0, p = 1, a = 5 - 20, bevorzugt 7 - 15, besonders bevorzugt 8 - 11, c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Isopropyl-Gruppen (-CH(CH₃)₂), R² = -C_{c}H_{2c}-O-(C₂H₄O-)ₐR⁵, R⁵ = ein Wasserstoffatom,
n = 0, p = 1, a = 7, 8, 9, 10, 11, 12, 13, 14 oder 15, c = 3,
bevorzugt ausgewählt aus

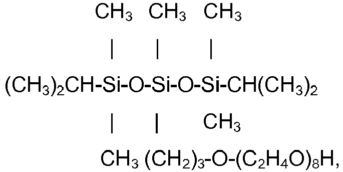

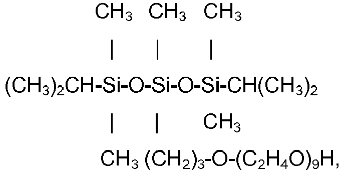

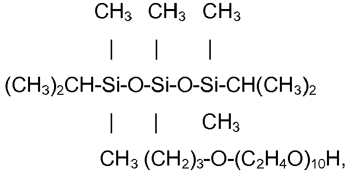

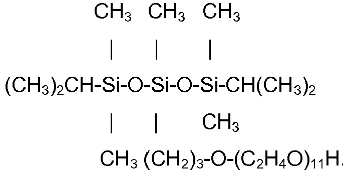

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methyl, R² = -CH₂-CH(CH₃)-CH₂-O-(C₂H₄O-)ₐR⁵,
R⁵ = ein Wasserstoffatom oder eine Methylgruppe,
n = 0, p = 1, a = 5 - 20, bevorzugt 7 - 15, besonders bevorzugt 8 - 11, c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methyl, R² = -CH₂-CH(CH₃)-CH₂-O-(C₂H₄O-)ₐR⁵, R⁵ = ein Wasserstoffatom,
n = 0, p = 1, a = 7, 8, 9, 10, 11, 12, 13, 14 oder 15, c = 3,
bevorzugt ausgewählt aus

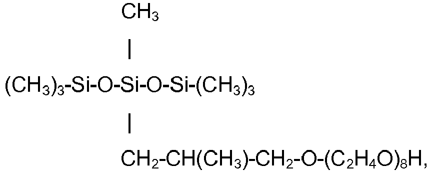

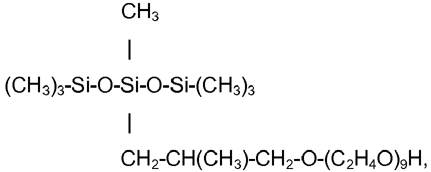

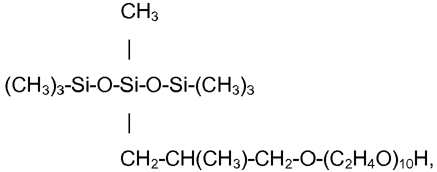

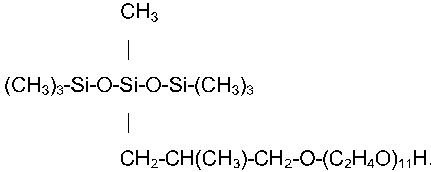

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵,
R⁵ = ein Wasserstoffatom oder eine Methylgruppe,
n = 10 - 500, bevorzugt 20 - 400, besonders bevorzugt 50 - 300, p = 10 - 50,
a = 5 - 30, bevorzugt 10 - 25, besonders bevorzugt 22,
b = 5 - 30, bevorzugt 10 - 25, besonders bevorzugt 24,
c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵, R⁵ = ein Wasserstoffatom,
n = 10 - 500, bevorzugt 20 - 400, besonders bevorzugt 50 - 300,
p = 10 - 50, bevorzugt 15 - 40, besonders bevorzugt 20 - 30,
a = 5 - 30, bevorzugt 10 - 25, besonders bevorzugt 22,
b = 5 - 30, bevorzugt 10 - 25, besonders bevorzugt 24,
c = 3.

Weitere erfindungsgemäß besonders bevorzugte Zusammensetzungen sind dadurch gekennzeichnet, dass das Organosiloxan-Oxyalkylen-Copolymer ausgewählt ist aus Verbindungen der allgemeinen Strukturformel (II) mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵, R⁵ = ein Wasserstoffatom,
n = 10 - 500, bevorzugt 20 - 400, besonders bevorzugt 50 - 300,
p = 10 - 50, bevorzugt 15 - 40, besonders bevorzugt 20 - 30,
a = 5 - 30, bevorzugt 10 - 25, besonders bevorzugt 22, b = 5 - 30, c = 3.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 18, b = 18, c = 3, R⁵ = Methyl, n = 10 - 500, p = 10 - 50.

Ein derartiges Organosiloxan-Oxyalkylen-Copolymer ist beispielsweise unter der Handelsbezeichnung Dow Corning 190 (INCI: PEG/PPG-18/18 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 12, b = 0, c = 3, R⁵ = Methyl, n = 10 - 500, p = 10 - 50.

Ein derartiges Organosiloxan-Oxyalkylen-Copolymer ist beispielsweise unter der Handelsbezeichnung Dow Corning 193 (INCI: PEG-12 Dimethicone) erhältlich.

Unter bestimmten Bedingungen kann Dow Corning 193 (PEG-12 Dimethicone) geruchsinstabil sein.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 7, b = 0, c = 2, R⁵ = Methyl, n = 0, p = 1.

Ein derartiges Organosiloxan-Oxyalkylen-Copolymer ist beispielsweise unter der Handelsbezeichnung Silwet L-77 erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 22, b = 24, c = 3, R⁵ = Methyl, n = 10 - 500, p = 10 - 50
= -(CH₂)₃-O-(C₂H₄O-)₂₂(C₃H₆O-)₂₄CH₃.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 17, b = 18, c = 3, R⁵ = Methyl, n = 10 - 500, p = 10 - 50.

Ein derartiges Organosiloxan-Oxyalkylen-Copolymer ist beispielsweise unter der Handelsbezeichnung Dow Corning Q2-5220 (INCI: PEG/PPG-17/18 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 20, b = 6, c = 3, R⁵ = Methyl, n = 10 - 500, p = 5 - 50.

Ein derartiges Organosiloxan-Oxyalkylen-Copolymer ist beispielsweise unter der Handelsbezeichnung Abil B 88184 (INCI: PEG/PPG-20/6 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 14, b = 4, c = 3, R⁵ = Methyl, n = 10 - 500, p = 5 - 50.

Ein derartiges Organosiloxan-Oxyalkylen-Copolymer ist beispielsweise unter der Handelsbezeichnung Abil B 8851 (INCI: PEG/PPG-14/4 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = tert.-Butyl, R² = -C_{c}H_{2c}-O-(C₂H₄O-)ₐ(C₃H₆O-)_{b}R⁵
mit a = 11, b = 0, c = 3, R⁵ = H, n = 0, p = 1.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Isopropyl (-CH(CH₃)₂), R² = -C_{c}H_{2c}-O-(C₂H₄O-)ₐ(C₃H₆O-)_{b}R⁵
mit a = 11, b = 0, c = 3, R⁵ = H, n = 0, p = 1.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit einem HLB-Wert im Bereich von 8 - 20, bevorzugt 10 - 18, besonders bevorzugt 11 - 16, und mit
R¹ = Methyl, R² = -CH₂-CH(CH₃)-CH₂-O-(C₂H₄O-)ₐ(C₃H₆O-)_{b}R⁵
mit a = 8, b = 0, R⁵ = H, n = 0, p = 1.

Weitere bevorzugte erfindungsgemäße Zusammensetzungen sind dadurch gekennzeichnet, dass mindestens ein Organosiloxan-Oxyalkylen-Copolymer der allgemeinen Strukturformel (I) enthalten ist mit einem HLB-Wert im Bereich von 8 - 20, bevorzugt 10 - 18, besonders bevorzugt 11 - 16, und mit
R¹ = Methyl, R² = -C_{c}H_{2c}-O-(C₂H₄O-)ₐ(C₃H₆O-)_{b}R⁵
mit a = 20, b = 20, c = 3, R⁵ = Methyl, m = 0, n = 10 - 500.

Ein derartiges Organosiloxan-Oxyalkylen-Copolymer ist beispielsweise unter der Handelsbezeichnung Abil B 8832 (INCI: Bis-PEG/PPG-20/20 Dimethicone) erhältlich.

Ein weiteres bevorzugtes ist Organosiloxan-Oxyalkylen-Copolymer ist Bis-PEG/PPG-16/16 PEG/PPG-16/16 Dimethicone.

Besonders bevorzugt sind die oben explizit angeführten Organosiloxan-Oxyalkylen-Copolymere der allgemeinen Strukturformel (II), die gegenüber den Organosiloxan-Oxyalkylen-Copolymeren der allgemeinen Strukturformel (I) eine bessere Wirkstofffreisetzung bewirken.

Weitere erfindungsgemäß besonders bevorzugte Organosiloxan-Oxyalkylen-Copolymere sind ausgewählt aus linearen Polysiloxan-Polyoxyalkylen-Blockcopolymeren, insbesondere aus linearen Polysiloxan-Polyoxyethylen-Polyoxypropylen-Blockcopolymeren. Erfindungsgemäß außerordentlich bevorzugt ist ein lineares Polysiloxan-Polyoxyethylen-Polyoxypropylen-Blockcopolymer mit der INCI-Bezeichnung PEG/PPG-22/24 Dimethicone. Ein derartiges lineares Polysiloxan-Polyoxyethylen-Polyoxypropylen-Blockcopolymer ist beispielsweise unter der Handelsbezeichnung Mirasil DMCO (INCI: PEG/PPG-22/24 Dimethicone) von der Firma Rhodia erhältlich.

Ein weiteres bevorzugtes lineares Polysiloxan-Polyoxyethylen-Polyoxypropylen-Blockcopolymer dieses Typs trägt die INCI-Bezeichnung PEG/PPG-10/2 Dimethicone. Es ist beispielsweise unter der Handelsbezeichnung Mirasil DMCP 93 (INCI: PEG/PPG-10/2 Dimethicone) von der Firma Rhodia erhältlich

Weitere erfindungsgemäß besonders bevorzugte Zusammensetzungen sind dadurch gekennzeichnet, dass das Organosiloxan-Oxyalkylen-Copolymer ausgewählt ist aus PEG/PPG-18/18 Dimethicone, PEG-12 Dimethicone, PEG/PPG-22/24 Dimethicone, PEG/PPG-17/18 Dimethicone, PEG/PPG-20/6 Dimethicone, PEG/PPG-14/4 Dimethicone, Bis-PEG/PPG-16/16 PEG/PPG-16/16 Dimethicone sowie Mischungen hiervon.

Weitere erfindungsgemäß besonders bevorzugte Zusammensetzungen sind dadurch gekennzeichnet, dass sie PEG/PPG-22/24 Dimethicone und PEG-12 Dimethicone enthalten.

Weitere erfindungsgemäß besonders bevorzugte Zusammensetzungen sind dadurch gekennzeichnet, dass sie PEG/PPG-22/24 Dimethicone und PEG/PPG-20/6 Dimethicone enthalten.

Weitere erfindungsgemäß besonders bevorzugte Zusammensetzungen sind dadurch gekennzeichnet, dass sie PEG/PPG-22/24 Dimethicone und PEG/PPG-14/4 Dimethicone enthalten.

Weitere erfindungsgemäß besonders bevorzugte Zusammensetzungen sind dadurch gekennzeichnet, dass sie PEG/PPG-22/24 Dimethicone und PEG/PPG-17/18 Dimethicone enthalten.

Weitere erfindungsgemäß besonders bevorzugte Zusammensetzungen sind dadurch gekennzeichnet, dass sie PEG/PPG-22/24 Dimethicone und Bis-PEG/PPG-20/20 Dimethicone enthalten.

Außerordentlich bevorzugt sind erfindungsgemäße Zusammensetzungen, die PEG/PPG-22/24 Dimethicone und PEG/PPG-20/6 Dimethicone enthalten.

Weiterhin außerordentlich bevorzugt sind erfindungsgemäße Zusammensetzungen, die PEG/PPG-22/24 Dimethicone und PEG/PPG-14/4 Dimethicone enthalten.

Weiterhin außerordentlich bevorzugt sind erfindungsgemäße Zusammensetzungen, die PEG/PPG-22/24 Dimethicone und PEG/PPG-17/18 Dimethicone enthalten.

Weitere erfindungsgemäß besonders bevorzugte Zusammensetzungen sind dadurch gekennzeichnet, dass sie PEG-12 Dimethicone und PEG/PPG-20/6 Dimethicone enthalten.

Weitere erfindungsgemäß besonders bevorzugte Zusammensetzungen sind dadurch gekennzeichnet, dass sie PEG-12 Dimethicone und PEG/PPG-14/4 Dimethicone enthalten.

Weitere erfindungsgemäß besonders bevorzugte Zusammensetzungen sind dadurch gekennzeichnet, dass sie PEG-12 Dimethicone und PEG/PPG-17/18 Dimethicone enthalten.

Weitere erfindungsgemäß besonders bevorzugte Zusammensetzungen sind dadurch gekennzeichnet, dass sie PEG-12 Dimethicone und Bis-PEG/PPG-20/20 Dimethicone enthalten.

Außerordentlich bevorzugt sind erfindungsgemäße Zusammensetzungen, die PEG-12 Dimethicone und PEG/PPG-20/6 Dimethicone enthalten.

Weiterhin außerordentlich bevorzugt sind erfindungsgemäße Zusammensetzungen, die PEG-12 Dimethicone und PEG/PPG-17/18 Dimethicone enthalten.

Weitere erfindungsgemäß besonders bevorzugte Zusammensetzungen sind dadurch gekennzeichnet, dass sie PEG/PPG-20/6 Dimethicone und PEG/PPG-14/4 Dimethicone enthalten.

Weitere erfindungsgemäß besonders bevorzugte Zusammensetzungen sind dadurch gekennzeichnet, dass sie PEG/PPG-20/6 Dimethicone und PEG/PPG-17/18 Dimethicone enthalten.

Weitere erfindungsgemäß besonders bevorzugte Zusammensetzungen sind dadurch gekennzeichnet, dass sie PEG/PPG-20/6 Dimethicone und Bis-PEG/PPG-20/20 Dimethicone enthalten.

Weitere erfindungsgemäß besonders bevorzugte Zusammensetzungen sind dadurch gekennzeichnet, dass sie PEG/PPG-14/4 Dimethicone und PEG/PPG-17/18 Dimethicone enthalten.

Weitere erfindungsgemäß besonders bevorzugte Zusammensetzungen sind dadurch gekennzeichnet, dass sie PEG/PPG-14/4 Dimethicone und Bis-PEG/PPG -20/20 Dimethicone enthalten.

Weitere erfindungsgemäß besonders bevorzugte Zusammensetzungen sind dadurch gekennzeichnet, dass sie PEG/PPG-17/18 Dimethicone und Bis-PEG/PPG-20/20 Dimethicone enthalten.

Weitere bevorzugte erfindungsgemäße Zusammensetzungen sind dadurch gekennzeichnet, dass das Organosiloxan-Oxyalkylen-Copolymer eine Wasserlöslichkeit von mindestens 2 g pro 100 g wässriger Lösung aufweist.

Besonders bevorzugte erfindungsgemäße Zusammensetzungen sind dadurch gekennzeichnet, dass das Organosiloxan-Oxyalkylen-Copolymer eine Wasserlöslichkeit von mindestens 5 g pro 100 g wässriger Lösung aufweist.

Weitere bevorzugte erfindungsgemäße Zusammensetzungen sind dadurch gekennzeichnet, dass das Organosiloxan-Oxyalkylen-Copolymer zu mindestens 2 Gew.-% mit Wasser mischbar ist.

Weitere besonders bevorzugte Zusammensetzungen sind dadurch gekennzeichnet, dass das Organosiloxan-Oxyalkylen-Copolymer zu mindestens 5 Gew.-% mit Wasser mischbar ist.

Alle vorstehenden Angaben zur Wasserlöslichkeit bzw. Wassermischbarkeit beziehen sich auf eine Temperatur von 20°C und einen Druck von 1013,25 mbar.

Weitere bevorzugte Zusammensetzungen enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine, und als dritte obligatorische Komponente das lineare Polysiloxan-Polyoxyethylen-Polyoxypropylen-Blockcopolymer PEG/PPG-22/24 Dimethicone. Die im Folgenden verwendete Bezeichnung "PEG/PPG-22/24 Dimethicone" meint immer das lineare Polysiloxan-Polyoxyethylen-Polyoxypropylen-Blockcopolymer PEG/PPG-22/24 Dimethicone.

Weitere bevorzugte Zusammensetzungen enthalten Triethylcitrat und eine Mischung aus Isodecan, Isoundecan, Isododecan und Isotridecan und das lineare Polysiloxan-Polyoxyethylen-Polyoxypropylen-Blockcopolymer PEG/PPG-22/24 Dimethicone.

Weitere bevorzugte Zusammensetzungen enthalten eine der folgenden Mischungen: Triethylcitrat/2-Ethylhexylpalmitat/PEG/PPG-22/24 Dimethicone, Triethylcitrat/Hexyldecyllaurat/ PEG/PPG-22/24 Dimethicone, Triethylcitrat/2-Ethylhexylstearat/PEG/PPG-22/24 Dimethicone, Triethylcitrat/Isopropylmyristat/PEG/PPG-22/24 Dimethicone, Triethylcitrat/Isopropylpalmitat/ PEG/PPG-22/24 Dimethicone, Triethylcitrat/2-Ethylhexyllaurat/PEG/PPG-22/24 Dimethicone, Triethylcitrat/C₁₂-C₁₅-Alkyllactat/PEG/PPG-22/24 Dimethicone, Triethylcitrat/C₁₂-C₁₅-Alkylbenzoat/ PEG/PPG-22/24 Dimethicone, Triethylcitrat/Di-C₁₂-C₁₃-Alkylmalat/PEG/PPG-22/24 Dimethicone.

Weiterhin sind Mischungen aus Triethylcitrat, mindestens einem flüchtigen C₈-C₁₆-Isoparaffin und mindestens einem Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, und PEG/PPG-22/24 Dimethicone bevorzugt. In derartigen Mischungen sind die Ethylester und die Isopropylester besonders bevorzugt; außerordentlich bevorzugt sind Isopropylpalmitat und Isopropylmyristat. Weiterhin sind Mischungen aus Triethylcitrat, mindestens einem flüchtigen C₈-C₁₆-Isoparaffin und mindestens einem Benzoesäureester von linearen oder verzweigten C₈₋₂₂-Alkanolen und PEG/PPG-22/24 Dimethicone bevorzugt.

Erfindungsgemäß besonders bevorzugte Mischungen sind Triethylcitrat/2-Ethylhexylpalmitat/Isodecan/Isoundecan/Isododecan/Isotridecan/PEG/PPG-22/24 Dimethicone, Triethylcitrat/Hexyldecyllaurat/Isodecan/Isoundecan/Isododecan/Isotridecan/PEG/PPG-22/24 Dimethicone, Triethyl-citrat/2-Ethylhexylstearat/Isodecan/Isoundecan/Isododecan/ Isotridecan/PEG/PPG-22/24 Dimethicone, Triethylcitrat/Isopropylmyristat/Isodecan/Isoundecan/Isododecan/Isotridecan/PEG/PPG-22/24 Dimethicone, Triethylcitrat/Isopropylpalmitat/Isononan/Isodecan/Isoundecan/Isododecan/ Isotridecan/PEG/PPG-22/24 Dimethicone, Triethylcitrat/2-Ethylhexyllaurat/Isodecan/Isoundecan/ Isododecan/Isotridecan/PEG/PPG-22/24 Dimethicone, Triethylcitrat/C₁₂-C₁₅-Alkyllactat/Isodecan/ Isoundecan/Isododecan/Isotridecan/PEG/PPG-22/24 Dimethicone, Triethylcitrat/C₁₂-C₁₅-Alkylbenzoat/Isodecan/Isoundecan/Isododecan/Isotridecan/PEG/PPG-22/24 Dimethicone und Triethylcitrat/Di-C₁₂-C₁₃-Alkylmalat/Isodecan/Isoundecan/Isododecan/Isotridecan/PEG/PPG-22/24 Dimethicone.

Weitere bevorzugte Zusammensetzungen enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine, und als dritte obligatorische Komponente das lineare Polysiloxan-Polyoxyethylen-Polyoxypropylen-Blockcopolymer PEG/PPG-10/2 Dimethicone. Die im Folgenden verwendete Bezeichnung "PEG/PPG-10/2 Dimethicone" meint immer das lineare Polysiloxan-Polyoxyethylen-Polyoxypropylen-Blockcopolymer PEG/PPG-10/2 Dimethicone.

Weitere bevorzugte Zusammensetzungen enthalten Triethylcitrat und eine Mischung aus Isodecan, Isoundecan, Isododecan und Isotridecan und das lineare Polysiloxan-Polyoxyethylen-Polyoxypropylen-Blockcopolymer PEG/PPG-10/2 Dimethicone.

Weitere bevorzugte Zusammensetzungen enthalten eine der folgenden Mischungen: Triethylcitrat/2-Ethylhexylpalmitat/PEG/PPG-10/2 Dimethicone, Triethylcitrat/Hexyldecyllaurat/ PEG/PPG-10/2 Dimethicone, Triethylcitrat/2-Ethylhexylstearat/PEG/PPG-10/2 Dimethicone, Tri-ethylcitrat/Isopropylmyristat/PEG/PPG-10/2 Dimethicone, Triethylcitrat/Isopropylpalmitat/PEG/ PPG-10/2 Dimethicone, Triethylcitrat/2-Ethylhexyllaurat/PEG/PPG-10/2 Dimethicone, Triethyl-citrat/C₁₂-C₁₅-Alkyllactat/PEG/PPG-10/2 Dimethicone, Triethylcitrat/C₁₂-C₁₅-Alkylbenzoat/PEG/ PPG-10/2 Dimethicone, Triethylcitrat/Di-C₁₂-C₁₃-Alkylmalat/PEG/PPG-10/2 Dimethicone.

Weiterhin sind Mischungen aus Triethylcitrat, mindestens einem flüchtigen C₈-C₁₆-Isoparaffin und mindestens einem Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, und PEG/PPG-10/2 Dimethicone bevorzugt. In derartigen Mischungen sind die Ethylester und die Isopropylester besonders bevorzugt; außerordentlich bevorzugt sind Isopropylpalmitat und Isopropylmyristat. Weiterhin sind Mischungen aus Triethylcitrat, mindestens einem flüchtigen C₈-C₁₆-Isoparaffin und mindestens einem Benzoesäureester von linearen oder verzweigten C₈₋₂₂-Alkanolen und PEG/PPG-10/2 Dimethicone bevorzugt.

Erfindungsgemäß besonders bevorzugte Mischungen sind Triethylcitrat/2-Ethylhexylpalmitat/Isodecan/Isoundecan/Isododecan/Isotridecan/PEG/PPG-10/2 Dimethicone, Triethylcitrat/Hexyldecyllaurat/Isodecan/Isoundecan/Isododecan/Isotridecan/PEG/PPG-10/2 Dimethicone, Triethylcitrat/2-Ethylhexylstearat/Isodecan/Isoundecan/Isododecan/Isotridecan/PEG/PPG-10/2 Dimethicone, Triethylcitrat/Isopropylmyristat/Isodecan/Isoundecan/Isododecan/Isotridecan/PEG/PPG-10/2 Dimethicone, Triethylcitrat/Isopropylpalmitat/Isononan/Isodecan/Isoundecan/Isododecan/Isotridecan/PEG/PPG-10/2 Dimethicone, Triethylcitrat/2-Ethylhexyllaurat/Isodecan/Isoundecan/Isododecan/Isotridecan/PEG/PPG-10/2 Dimethicone, Triethylcitrat/C₁₂-C₁₅-Alkyllactat/Isodecan/Isoundecan/Isododecan/Isotridecan/PEG/PPG-10/2 Dimethicone, Triethylcitrat/C₁₂-C₁₅-Alkylbenzoat/ Isodecan/Isoundecan/Isododecan/Isotridecan/PEG/PPG-10/2 Dimethicone und Triethylcitrat/ Di-C₁₂-C₁₃-Alkylmalat/Isodecan/Isoundecan/Isododecan/Isotridecan/PEG/PPG-10/2 Dimethicone. Weitere bevorzugte erfindungsgemäße Zusammensetzungen enthalten mindestens eines der vorstehend bezeichneten Organosiloxan-Oxyalkylen-Copolymere in einer Gesamtmenge von 0,01 - 5 Gew.-%, bevorzugt 0,1 - 3 Gew.-%, besonders bevorzugt 0,5 - 2 Gew.-%, außerordentlich bevorzugt 0,7 - 1,5 Gew.-%, jeweils bezogen auf das Gesamtgewicht der treibmittelfreien Zusammensetzung. Der Anteil der Organosiloxan-Oxyalkylen-Copolymere wird bei der Berechnung des Gewichtsanteils von Triethylcitrat an der Gesamtmenge an Ölen c) + d) + e) nicht berücksichtigt.

Die schweißhemmenden Wirkstoffe und gegebenenfalls weitere im Träger unlösliche Wirkstoffe sind in unter Normalbedingungen flüssigen Ölmischung c) + d) (+ ggf. e)) suspendiert. Zur besseren Anwendbarkeit wird dieser Suspension bevorzugt mindestens ein lipophiles Verdickungsmittel als Suspendierhilfe zugesetzt. Weitere bevorzugte erfindungsgemäße Zusammensetzungen sind daher dadurch gekennzeichnet, dass sie mindestens ein lipophiles Verdickungsmittel enthalten.

Bevorzugte erfindungsgemäße Zusammensetzungen sind dadurch gekennzeichnet, dass das mindestens eine lipophile Verdickungsmittel ausgewählt ist aus hydrophobierten Tonmineralien, pyrogenen Kieselsäuren, Bentone-Gelen, Ethylene/Propylene/Styrene Copolymeren, Butylene/ Ethylene/Styrene Copolymeren, Dextrinestern, Siliconelastomeren, unter Normalbedingungen festen Wachsen und/oder Glycerintriestern. Hierunter sind hydrophobierte Tonmineralien besonders bevorzugt. Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien als Pulver oder in Form eines Gels in einer Ölkomponente bereit. Derartige Pulver oder Gele sind beispielsweise unter der Handelsbezeichnung Bentone^{®} oder Thixogel erhältlich.

Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der treibmittelfreien Zusammensetzung, enthalten.

Derartige hydrophobierte Tonmineralien benötigen üblicherweise als Aktivator Wasser, Ethanol oder Propylencarbonat in einer Menge von 0,3 - 3 Gew.-%, bevorzugt 0,5 - 2 Gew.-%, jeweils bezogen auf das Gesamtgewicht der treibmittelfreien erfindungsgemäßen Zusammensetzung.

Weitere erfindungsgemäß bevorzugte lipophile Verdickungsmittel sind ausgewählt aus pyrogenen Kieselsäuren, z. B. den Handelsprodukten der Aerosil^{®}-Serie von Evonik Degussa. Besonders bevorzugt sind hydrophobierte pyrogene Kieselsäuren, außerordentlich bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine hydrophobierte pyrogene Kieselsäure, in einer Gesamtmenge von 0,5 - 10 Gew.-%, bevorzugt 0,8 - 5 Gew.-%, besonders bevorzugt 1 - 4 Gew.-%, außerordentlich bevorzugt 1,5 - 2 Gew.-%, jeweils bezogen auf das Gesamtgewicht der treibmittelfreien erfindungsgemäßen Zusammensetzung, enthalten.

Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Die erfindungsgemäßen Zusammensetzungen enthalten mindestens einen Antitranspirant-Wirkstoff, der in ungelöster, suspendierter Form vorliegt.

Bevorzugte Antitranspirant-Wirkstoffe sind ausgewählt aus den wasserlöslichen adstringierenden anorganischen und organischen Salzen des Aluminiums und Zinks bzw. beliebigen Mischungen dieser Salze. Alumosilicate und Zeolithe zählen erfindungsgemäß nicht zu den Antitranspirant-Wirkstoffen.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat und Zinksulfat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.

Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu AlGewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.

Die schweißhemmenden Wirkstoffe liegen in ungelöster, suspendierter Form vor.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 - 15,5 µm, auf.

Bevorzugte Aluminiumsalze weisen ein molares Metall-zu-Chlorid-Verhältnis von 1,9 -2,1, bzw. für Sesquichlorohydrate von 1,5:1-1,8:1 auf.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-%, besonders bevorzugt 11 - 28 Gew.-% und außerordentlich bevorzugt 12 - 20 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter dem Namen Reach^{®} 301 wird ein Aluminiumsesquichlorohydrat von Reheis angeboten, das auch besonders bevorzugt ist.

Die Konfektionierung der erfindungsgemäßen Zusammensetzungen, die als Spray appliziert werden, richtet sich vorzugsweise nach den Anforderungen der gewünschten Sprayapplikation.

Die erfindungsgemäßen Zusammensetzungen liegen als Suspension vor, das heißt, der schweißhemmende Wirkstoff und gegebenenfalls weitere unlösliche Bestandteile sind in einem flüssigen Träger suspendiert. Ein solches disperses System sollte vor der Anwendung geschüttelt werden.

In einer weiteren erfindungsgemäß bevorzugten Ausführungsform sind die erfindungsgemäßen Zusammensetzungen als mit einem Treibmittel versprühbare Suspension konfektioniert.

Bevorzugte erfindungsgemäße Zusammensetzungen können z. B. in Pump- oder Quetschspendern abgepackt sein, insbesondere in Mehrkammer-Pump- oder Quetschspendern. Derartige Spender verwenden Luft, insbesondere die Umgebungsluft, als Treibmittel bzw. fördern die erfindungsgemäße Zusammensetzung durch Pumpen.

In einer weiteren bevorzugten Ausführungsform der Erfindung wird die Zusammensetzung mittels eines komprimierten bzw. verflüssigten Treibmittels appliziert.

Alle Mengenangaben beziehen sich, sofern nicht anders angegeben, auf das Gewicht der treibmittelfreien Zusammensetzung.

Die Verpackung in einem Mehrkammerspender bietet besondere technische Vorteile.

Der Mehrkammerspender kann auch so eingesetzt werden, dass eine Kammer mit der erfindungsgemäßen Zusammensetzung befüllt ist, während eine andere Kammer das komprimierte Treibmittel enthält. Ein derartiger Mehrkammerspender ist beispielsweise eine so genannte Bagin-Can-Verpackung.

Beide Kammern können aber auch so miteinander verbunden, dass die erfindungsgemäße Zusammensetzung in zwei Teilzusammensetzungen aufgetrennt wird, die gleichzeitig aus der Verpackung ausgegeben werden können, beispielsweise aus getrennten Öffnungen oder aus einer einzigen Öffnung.

Weitere bevorzugte erfindungsgemäße Zusammensetzungen sind dadurch gekennzeichnet, dass sie mit mindestens einem Treibmittel in einem geeigneten Druckbehälter verpackt sind.

Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Besonders bevorzugt sind Propan, n-Butan, iso-Butan sowie, besonders bevorzugt, Mischungen dieser Treibmittel.

Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie insbesondere Mischungen dieser Treibgase.

Die Menge der Treibmittel beträgt bevorzugt 20 - 95 Gew.-%, besonders bevorzugt 30 - 90 Gew.-% und außerordentlich bevorzugt 60 - 86 Gew.-%, und weiterhin außerordentlich bevorzugt 75 - 78 Gew.-%, jeweils bezogen auf das Gesamtgewicht der Zubereitung, bestehend aus der erfindungsgemäßen Suspension und dem Treibmittel.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Befüllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Suspension. Ein erfindungsgemäß bevorzugter Innenschutzlack ist ein Epoxy-Phenollack, wie er u.a. unter dem Namen Hoba 7407 P erhältlich ist. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Weitere bevorzugte erfindungsgemäße Zusammensetzungen sind dadurch gekennzeichnet, dass sie mindestens einen Duftstoff enthalten. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen sind dadurch gekennzeichnet, dass mindestens ein Duftstoff in einer Gesamtmenge von 0,1 - 10 Gew.-%, bevorzugt 0,2 - 7 Gew.-%, besonders bevorzugt 0,4 - 6 Gew.-%, außerordentlich bevorzugt 1 - 5 Gew.-%, weiterhin außerordentlich bevorzugt 2 - 4 Gew.-%, jeweils bezogen auf das Gesamtgewicht der treibmittelfreien Zusammensetzung, enthalten ist.

Weitere erfindungsgemäß bevorzugte Zusammensetzungen sind gekennzeichnet durch einen Gehalt an mindestens einem so genannten "hautkühlenden Wirkstoff". Unter hautkühlenden Wirkstoffen im Sinne der vorliegenden Anmeldung werden Wirkstoffe verstanden, die bei Applikation auf die Haut infolge Oberflächenanästhesierung und Reizung der kälteempfindlichen Nerven bei Migräne und dergleichen ein angenehmes Kältegefühl erzeugen, auch wenn die behandelten Hautpartien tatsächlich normale bzw. erhöhte Temperatur zeigen.

Bevorzugte hautkühlende Wirkstoffe sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe besonders bevorzugt sind Menthol, Isopulegol, Menthyllactat, Menthoxypropandiol und Menthylpyrrolidoncarbonsäure.

Bevorzugte erfindungsgemäße Zusammensetzungen enthalten mindestens einen hautkühlenden Wirkstoff in Gesamtmengen von 0,01 - 1,5 Gew.-%, bevorzugt 0,02 - 0,5 Gew.-% und besonders bevorzugt 0,05 - 0,2 Gew.-%, jeweils bezogen auf das Gesamtgewicht der (treibmittelfreien) Zusammensetzung.

Bevorzugte erfindungsgemäße Zusammensetzungen sind dadurch gekennzeichnet, dass mindestens ein verkapselter Wirkstoff enthalten ist. Die Wirkstoffe, die vorteilhafterweise verkapselt sein können, sind insbesondere Duftstoffe, Parfümöle und/oder hautkühlende Wirkstoffe, aber auch andere hautpflegende Wirkstoffe, wie Vitamine, Antioxidantien etc.

Als Kapselmaterial bevorzugt sind wasserlösliche Polymere wie Stärke, physikalisch modifizierte und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren.

Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Capsul von National Starch, des weiteren Carboxymethylcellulose, Carboxymethylcellulose, Methylcellulose, Hydroxyethylcellulose und Hydroxypropylmethylcellulose, Ethylcellulose, z. B. das Handelsprodukt Tylose H 10 von Clariant, weiterhin Carragheene, Alginate und Maltodextrine, sowie beliebige Mischungen dieser Polymere.

Die erfindungsgemäßen Zusammensetzungen enthalten aus Gründen des Korrosionsschutzes 0 bis 3 Gew.-% Ethanol, bezogen auf die gesamte treibmittelfreie Zusammensetzung. Bevorzugt sind Ethanol-Gehalte von 0 - 1 Gew.-%.

Die erfindungsgemäßen Zusammensetzungen sind im Wesentlichen wasserfrei, d. h. sie enthalten 0 bis maximal 5 Gew.-%, bevorzugt 0,5 bis 4 Gew.-%, besonders bevorzugt 1 bis 3 Gew.-%, außerordentlich bevorzugt 1,5 bis 2,5 Gew.-% freies Wasser, jeweils bezogen auf die gesamte treibmittelfreie Zusammensetzung, wobei auch Gehalte an freiem Wasser von 1,6, 1,7, 1,8, 1,9, 2,0, 2,1, 2,2, 2,3 und 2,4 Gew.-% bevorzugt sein können. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar.

Weiterhin können die erfindungsgemäßen Zusammensetzungen zusätzliche Deodorantien enthalten. Als Deodorantien können antimikrobielle, antibakterielle oder keimhemmende Stoffe, Antioxidantien oder Geruchsadsorbentien (z. B. Zinkricinoleat) eingesetzt werden.

Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten, kolloidales, elementares Silber, anorganische oder organische Silbersalze, wie insbesondere Silbercitrat und Silberdihydrogencitrat, und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol kann als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Bevorzugte Deodorant-Wirkstoffe sind langkettige Diole, z. B. 1,2-Alkan-(C₅-C₁₈)-Diole, Glycerinmono(C₈-C₁₈)-Fettsäureester oder, besonders bevorzugt, Glycerinmono-(C₆-C₁₆)-alkylether, insbesondere 2-Ethylhexylglycerinether, die sehr gut haut- und schleimhautverträglich und gegen Corynebakterien wirksam sind, sowie weiterhin Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methyl-phenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethyl-propan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenyl-propan-1-ol und 2-n-Pentyl-3-phenylpropan-1-ol.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten.

Die Dosen sind mit einem geeigneten Sprühkopf ausgestattet. Je nach Sprühkopf sind Ausstoßraten, bezogen auf voll gefüllte Dosen, von 0,1 g/s bis 2,0 g/s möglich.

Unter "Verbesserung der Schweißreduktion" ist erfindungsgemäß sowohl eine Reduzierung der Schweißmenge als auch eine Beschleunigung der Freisetzung des schweißhemmenden Wirkstoffs aus der erfindungsgemäßen Zusammensetzung zu verstehen.

Ein weiterer Gegenstand der vorliegenden Anmeldung ist die nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs. Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7, 8, 9, 10, 11 oder 12 in einer wirksamen Menge auf die Haut, bevorzugt auf die Haut im Achselbereich, aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen die Erfindung verdeutlichen, ohne sie hierauf zu beschränken. Alle Mengenangaben sind in Gew.-%.

### Zur Erläuterung:

Mit dem Abfüllverhältnis ist das Gewichtsverhältnis von schweißhemmender Zusammensetzung (erfindungsgemäß oder Vergleichszusammensetzung) zu Treibmittel bezeichnet.

### Messung der Freisetzung des schweißhemmenden Wirkstoffs

Zum Nachweis, ob der schweißhemmende Wirkstoff schnell verfügbar ist, wurde der zeitliche Verlauf der Leitfähigkeit der Suspensionen aus einem definierten Film in einer bestimmten Menge entionisierten Wassers gemessen.

Der Wert für die am Ende des Versuchs erreichte Leitfähigkeit liegt für die erfindungsgemäßen Zusammensetzungen bei 80 - 160 Mikrosiemens [µS] pro Zentimeter. Die Zusammensetzungen des Standes der Technik wiesen eine finale Leitfähigkeit von maximal 10 - 50 Mikrosiemens pro Zentimeter auf.

Die Vergleichszusammensetzungen Nr. 1 bis Nr. 42 und die erfindungsgemäßen Zusammensetzungen wurden auf die Haut im Achselbereich aufgetragen.

Bei den erfindungsgemäßen Zusammensetzungen wurde eine schneller einsetzende schweißhemmende Wirkung beobachtet.

In den nachstehenden Tabellen ist eine qualitative Beurteilung der getesteten Zusammensetzungen vermerkt, mit einer fünfstufigen Skala von "sehr gut" bis "schlecht". In diese Beurteilung geht nicht nur der erreichte Endwert der Leitfähigkeit ein, sondern auch die Steigung der zeitlichen Veränderung der Leitfähigkeit zu Beginn des Versuchs. Eine starke Steigung wird als gleichbedeutend mit einer schnellen Freisetzung des schweißhemmenden Wirkstoffs interpretiert.

Die Zeile * bezeichnet den Gewichtsanteil von Triethylcitrat an der Gesamtölmenge c) plus d). Komponente e) = Cyclomethicone war nicht enthalten.

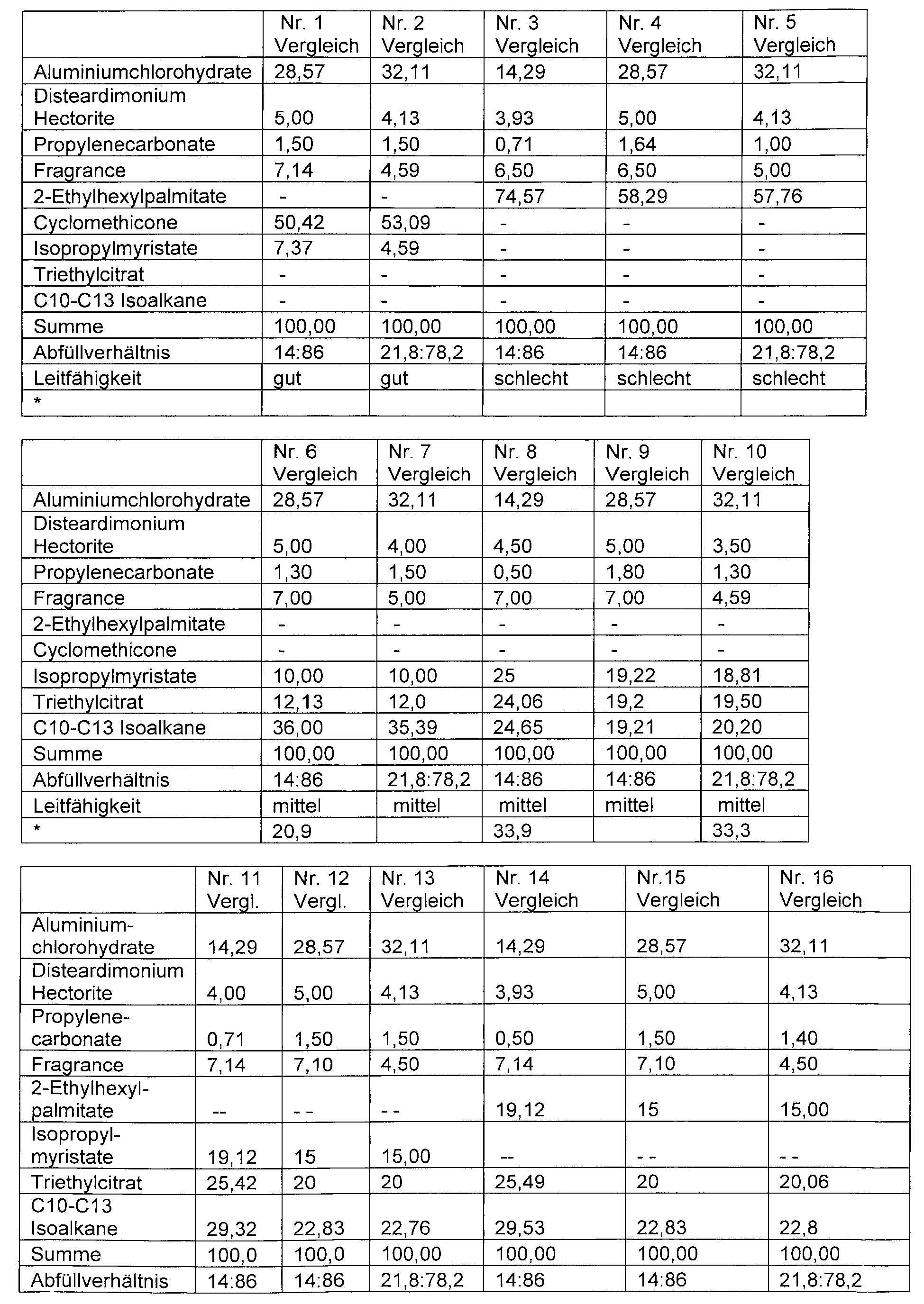

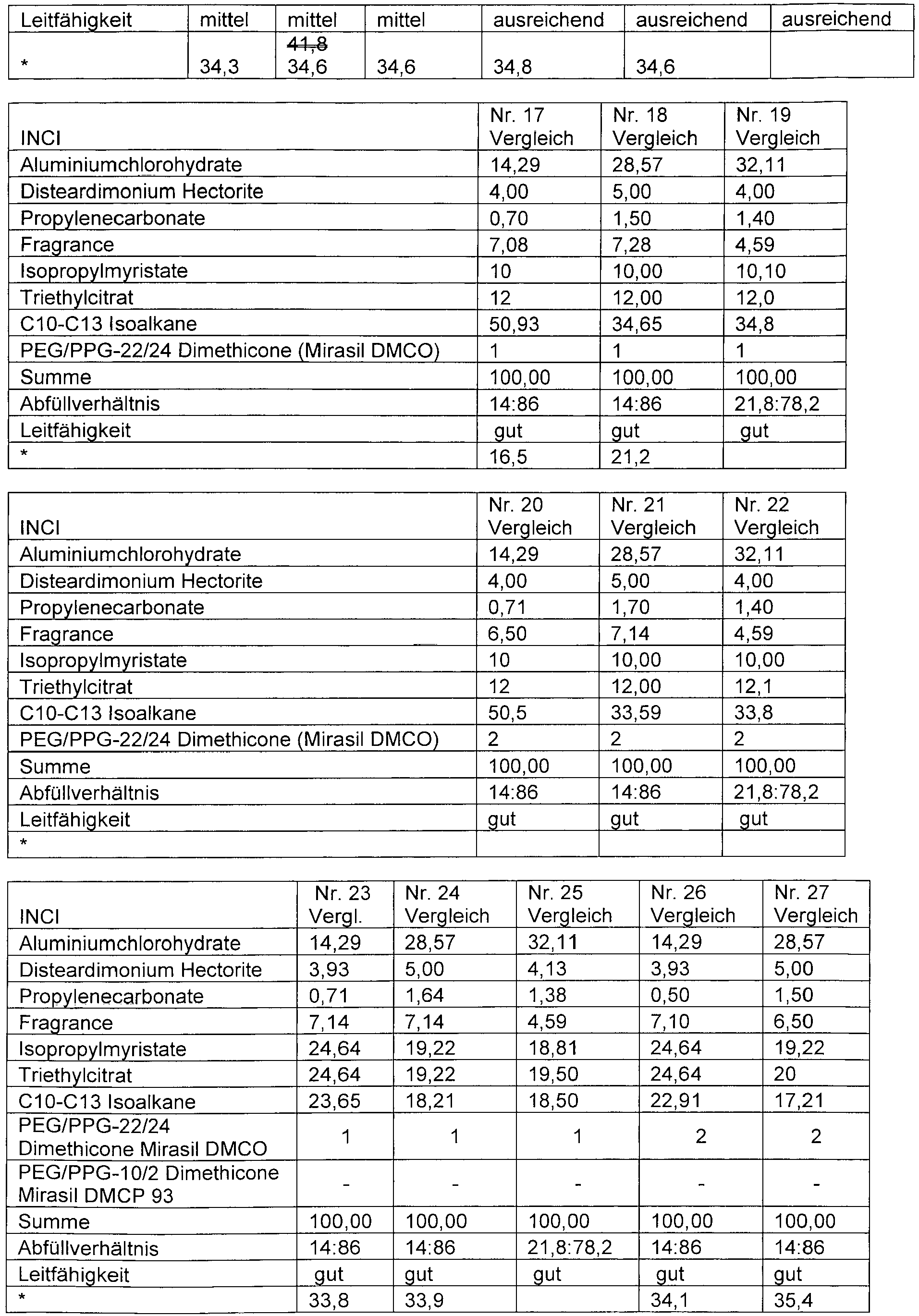

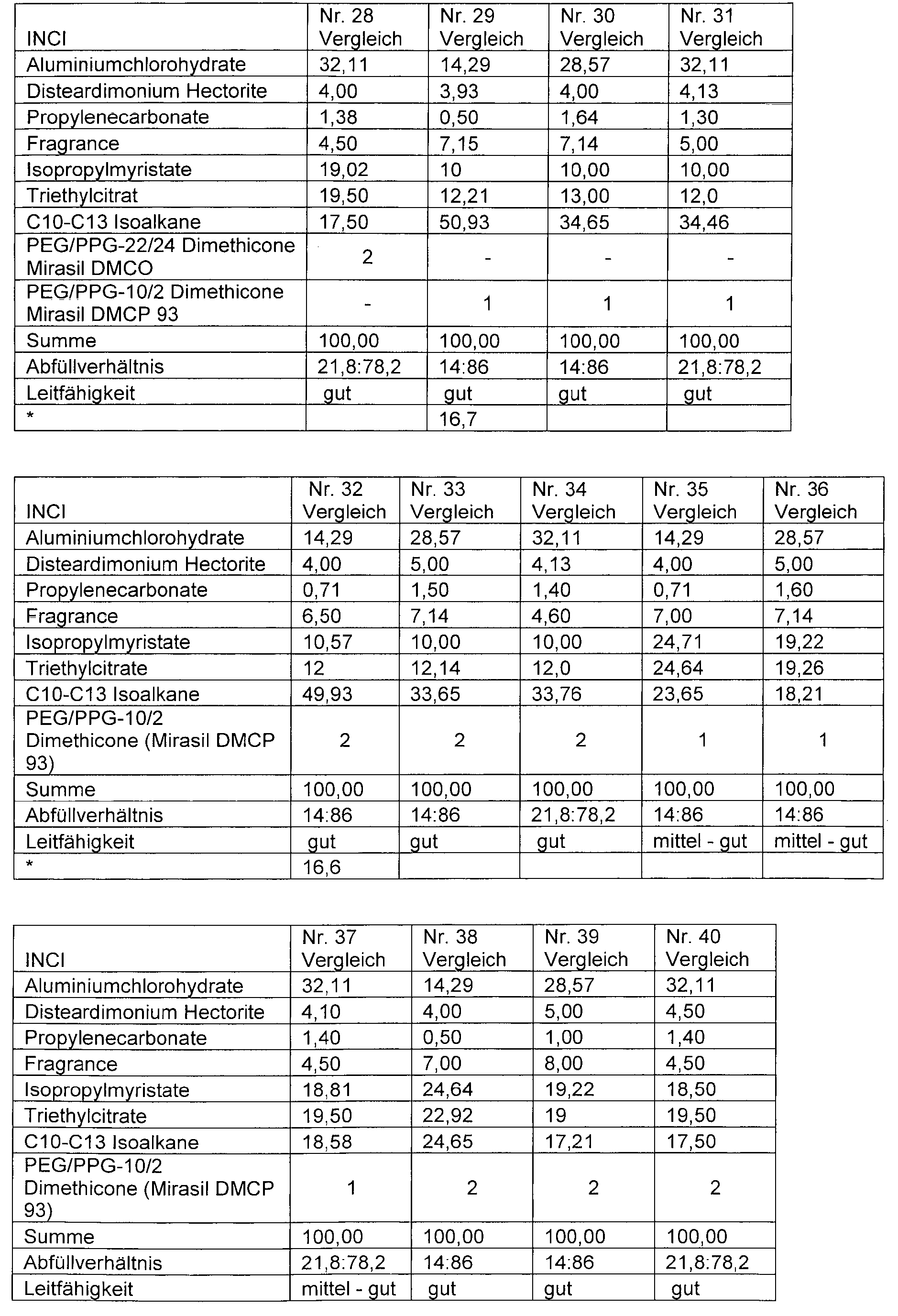

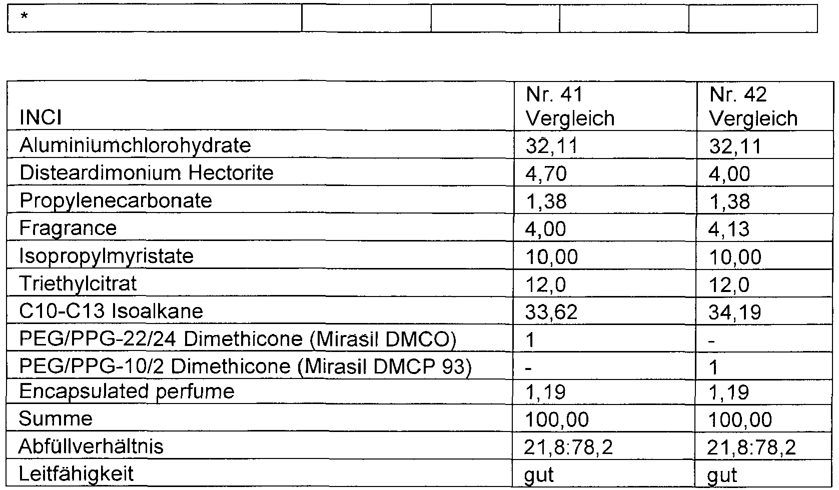

**Liste der verwendeten Rohstoffe**

| INCI-Bezeichnung | Rohstoffname | Hersteller/ Lieferant |
|---|---|---|
| Disteardimonium Hectorite | Bentone Powder 38 V CG | Elementis Specialities |
| PEG/PPG-22/24 Dimethicone | Mirasil DMCO (72 Gew.-% PEG/PPG-22/24 Dimethicone Aktivsubstanz), lineares Polysiloxan-Polyoxyalkylen-Blockcopolymer | Rhodia |
| PEG/PPG-10/2 Dimethicone | Mirasil DMCP 93 (93 Gew.-% PEG/PPG-10/2 Dimethicone Aktivsubstanz), lineares Polysiloxan-Polyoxyalkylen-Blockcopolymer | Rhodia |

## Patentansprüche

1. Schweißhemmende Zusammensetzung zur persönlichen Körperpflege, konfektioniert als treibmittelfrei versprühbare oder mit einem Treibmittel versprühbare Suspension, enthaltend
a) mindestens einen schweißhemmenden Wirkstoff, wobei die schweißhemmenden Wirkstoffe in ungelöster, suspendierter Form vorliegen,
b) 0 - 5 Gew.-%, freies Wasser, bezogen auf das Gewicht der treibmittelfreien Zusammensetzung,
c) Triethylcitrat,
d) mindestens ein weiteres unter Normalbedingungen flüssiges kosmetisches Öl als Träger,
e) 0 bis weniger als 1 Gew.-% Cyclomethicone, bezogen auf das Gewicht der treibmittelfreien Zusammensetzung,
f) 0 - 3 Gew.-% Ethanol, bezogen auf das Gewicht der treibmittelfreien Zusammensetzung,
**dadurch gekennzeichnet, dass** der Gewichtsanteil von Triethylcitrat an der Gesamtmenge an Ölen c) plus d) plus e) 40 - 50 Gew.-% beträgt.

2. Zusammensetzung gemäß Anspruch 1, **dadurch gekennzeichnet, dass** die Gesamtmenge an Triethylcitrat und mindestens einem weiteren, unter Normalbedingungen flüssigen kosmetischen Öl d) + e) 30 - 95 Gew.-%, bevorzugt 40 - 93 Gew.-%, besonders bevorzugt 50 - 90 Gew.-%, außerordentlich bevorzugt 53 - 63 Gew.-%, beträgt, jeweils bezogen auf die gesamte treibmittelfreie Zusammensetzung.

3. Zusammensetzung gemäß Anspruch 1 oder 2, **dadurch gekennzeichnet, dass** neben Triethylcitrat mindestens ein flüchtiges Nichtsiliconöl enthalten ist.

4. Zusammensetzung gemäß Anspruch 1, 2 oder 3, **dadurch gekennzeichnet, dass** das mindestens eine unter Normalbedingungen flüssige Träger-Öl d), das ein flüchtiges Nichtsiliconöl ist, ausgewählt ist aus C₈-C₁₆-Isoparaffinen, insbesondere aus Isononan, Isodecan, Isoundecan, Isododecan, Isotridecan, Isotetradecan, Isopentadecan, und Isohexadecan, sowie Mischungen hiervon, wobei C₁₀-C₁₃-Isoparaffin-Mischungen besonders bevorzugt sind.

5. Zusammensetzung gemäß Anspruch 4, **dadurch gekennzeichnet, dass** das mindestens eine C₈-C₁₆-Isoparaffin in einer Gesamtmenge von 25 - 50 Gew.-%, bevorzugt 30 - 45 Gew.-%, besonders bevorzugt 32 - 40 Gew.-%, außerordentlich bevorzugt 33, 34, 35, 36, 37, 38 oder 39 Gew.-%, jeweils bezogen auf die gesamte treibmittelfreie Zusammensetzung, enthalten ist.

6. Zusammensetzung gemäß Anspruch 1, 2, 3, 4 oder 5, **dadurch gekennzeichnet, dass** neben Triethylcitrat und dem mindestens einen C₈-C₁₆-Isoparaffin mindestens ein nichtflüchtiges kosmetisches Öl, ausgewählt aus nichtflüchtigen Siliconölen und nichtflüchtigen Nichtsiliconölen, enthalten ist.

7. Zusammensetzung gemäß Anspruch 1, 2, 3, 4, 5 oder 6, **dadurch gekennzeichnet, dass** das mindestens eine nichtflüchtige Nichtsiliconöl ausgewählt ist aus Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, sowie aus den Benzoesäureestern von linearen oder verzweigten C₈₋₂₂-Alkanolen, bevorzugt ausgewählt aus Isopropylpalmitat, Isopropylmyristat, Isopropylstearat, Hexyldecylstearat, Hexyldecyllaurat, Isononylisononanoat, 2-Ethylhexylpalmitat, 2-Ethylhexylstearat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat, Di-C₁₂-C₁₃-Alkylmalat, C₁₂-C₁₅-Alkylbenzoat, Benzoesäureisostearylester, Ethylhexylbenzoat und Benzoesäureoctyldocecylester,

8. Zusammensetzung gemäß einem der Ansprüche 1, 2, 3, 4, 5, 6 oder 7, **dadurch gekennzeichnet, dass** die Komponente d) ausgewählt ist aus mindestens einer Mischung aus d)i) mindestens einem flüchtigen C₈-C₁₆-Isoparaffin und d)ii) mindestens einem Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, sowie aus mindestens einer Mischung aus d)i) mindestens einem flüchtigen C₈-C₁₆-Isoparaffin und d)ii) mindestens einem Benzoesäureester von linearen oder verzweigten C₈₋₂₂-Alkanolen.

9. Zusammensetzung gemäß einem der Ansprüche 1, 2, 3, 4, 5, 6, 7 oder 8, **dadurch gekennzeichnet, dass** Triethylcitrat/Ester/C₁₀-C₁₃-Isoparaffin in Gewichtsverhältnissen Triethylcitrat/ Ester/C₁₀₋₁₃-Isoparaffin von (1-1,3) : (0,6-1) : (1-3), bevorzugt (1-1,3) : 1 : (1-1,5), ebenfalls bevorzugt (1-1,3) : (0,6 - 0,9) : (2,5 - 3), insbesondere 1 : 0,8 : 3, enthalten sind.

10. Zusammensetzung gemäß einem der Ansprüche 1, 2, 3, 4, 5, 6, 7, 8 oder 9, **dadurch gekennzeichnet, dass** mindestens ein Organosiloxan-Oxyalkylen-Copolymer enthalten ist, das ausgewählt ist aus einem linearen Polysiloxan-Polyoxyethylen-Polyoxypropylen-Blockcopolymer mit der INCI-Bezeichnung PEG/PPG-22/24 Dimethicone und einem linearen Polysiloxan-Polyoxyethylen-Polyoxypropylen-Blockcopolymer mit der INCI-Bezeichnung PEG/PPG-10/2 Dimethicone sowie Mischungen hiervon.

11. Zusammensetzung gemäß Anspruch 10, **dadurch gekennzeichnet, dass** das mindestens eine Organosiloxan-Oxyalkylen-Copolymer in einer Gesamtmenge von 0,01 - 5 Gew.-%, bevorzugt 0,1 - 3 Gew.-%, besonders bevorzugt 0,5 - 2 Gew.-%, außerordentlich bevorzugt 0,7 - 1,5 Gew.-%, enthalten ist, jeweils bezogen auf das Gesamtgewicht der treibmittelfreien Zusammensetzung.

12. Zusammensetzung gemäß einem der Ansprüche 1, 2, 3, 4, 5, 6, 7, 8, 9, 10 oder 11, **dadurch gekennzeichnet, dass** die Ölmischung c) plus d) ausgewählt ist aus Triethylcitrat/ 2-Ethyl-hexylpalmitat/Isodecan/Isoundecan/Isododecan/Isotridecan, Triethylcitrat/Hexyldecyl-laurat/Isodecan/Isoundecan/Isododecan/Isotridecan, Triethylcitrat/2-Ethylhexylstearat/Isodecan/Isoundecan/Isododecan/Isotridecan, Triethylcitrat/Isopropylmyristat/Isodecan/Isoundecan/Isododecan/Isotridecan, Triethylcitrat/Isopropylpalmitat/Isononan/Isodecan/Isoundecan/ Isododecan/Isotridecan, Triethylcitrat/ 2-Ethylhexyllaurat/Isodecan/Isoundecan/Isododecan/ Isotridecan, Triethylcitrat/C₁₂-C₁₅-Alkyllactat/ Isodecan/Isoundecan/Isododecan/Isotridecan, Triethylcitrat/C₁₂-C₁₅-Alkylbenzoat/Isodecan/Isoundecan/Isododecan/Isotridecan und Triethylcitrat/Di-C₁₂-C₁₃-Alkylmalat/Isodecan/Isoundecan/Isododecan/Isotridecan.

13. Nicht-therapeutisches, kosmetisches Verfahren zur Reduzierung und/oder Regulierung der Schweißbildung und/oder des Körpergeruchs, bei dem eine Zusammensetzung gemäß einem der Ansprüche 1, 2, 3, 4, 5, 6, 7, 8, 9, 10, 11 oder 12 in einer wirksamen Menge auf die Haut, bevorzugt auf die Haut im Achselbereich, aufgetragen wird.

14. Nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs.

## Claims

1. An antiperspirant composition for personal hygiene, formulated as a suspension which is sprayable with or without a propellant, containing:
a) at least one antiperspirant active ingredient, the antiperspirant active ingredients being present in an undissolved, suspended form,
b) from 0-5 wt. % of free water, based on the weight of the propellant-free composition,
c) triethyl citrate,
d) at least one further cosmetic oil, which is liquid under normal conditions, as the carrier,
e) from 0 to less than 1 wt. % of cyclomethicone, based on the weight of the propellant-free composition,
f) from 0-3 wt.% ethanol, based on the weight of the propellant-free composition,
**characterized in that** the proportion by weight of triethyl citrate in the total quantity of oils c) plus d) plus e) is from 40-50 wt. %.

2. The composition according to claim 1, **characterized in that** the total quantity of triethyl citrate and at least one further cosmetic oil, which is liquid under normal conditions, d)+e) is from 30-95 wt. %, preferably from 40-93 wt.%, particularly preferably from 50-90 wt.%, most preferably from 53-63 wt.%, in each case based on the total propellant-free composition.

3. The composition according to claim 1 or claim 2, **characterized in that**, in addition to triethyl citrate, said composition contains at least one volatile non-silicone oil.

4. The composition according to claim 1, 2 or 3, **characterized in that** the at least one carrier oil d), which is liquid under normal conditions, is a volatile non-silicone oil selected from C₈-C₁₆ isoparaffins, in particular from isononane, isodecane, isoundecane, isododecane, isotridecane, isotetradecane, isopentadecane, and isohexadecane, and mixtures thereof, C₁₀-C₁₃ isoparaffin mixtures being particularly preferable.

5. The composition according to claim 4, **characterized in that** it contains the at least one C₈-C₁₆ isoparaffin in a total quantity of from 25-50 wt. %, preferably from 30-45 wt.%, particularly preferably from 32-40 wt.%, most preferably 33, 34, 35, 36, 37, 38 or 39 wt.%, in each case based on the total propellant-free composition.

6. The composition according to claim 1, 2, 3, 4 or 5, **characterised in that**, in addition to triethyl citrate and the at least one C₈-C₁₆ isoparaffin, said composition contains at least one non-volatile cosmetic oil selected from non-volatile silicone oils and non-volatile non-silicone oils.

7. The composition according to claim 1, 2, 3, 4, 5 or 6, **characterized in that** the at least one non-volatile non-silicone oil is selected from esters of linear or branched, saturated or unsaturated fatty alcohols having 2-30 carbon atoms with linear or branched, saturated or unsaturated fatty acids having 2-30 carbon atoms, which may be hydroxylated, and from benzoic acid esters of linear or branched C₈₋₂₂ alkanols, preferably selected from isopropyl palmitate, isopropyl myristate, isopropyl stearate, hexyldecyl stearate, hexyldecyl laurate, isononyl isononanoate, 2-ethylhexyl palmitate, 2-ethylhexyl stearate, isooctyl stearate, isononyl stearate, isocetyl stearate, isononyl isononanoate, isotridecyl isononanoate, cetearyl isononanoate, 2-ethylhexyl laurate, 2-ethylhexyl isostearate, 2-ethylhexyl cocoate, 2-octyldodecyl palmitate, butyloctanoic acid 2-butyloctanoate, diisotridecyl acetate, n-hexyl laurate, n-decyl oleate, oleyl oleate, oleyl erucate, erucyl oleate, C₁₂-C₁₅ alkyl lactate, di-C₁₂-C₁₃-alkyl malate, C₁₂-C₁₅ alkyl benzoate, benzoic acid isostearyl ester, ethylhexyl benzoate and benzoic acid octyldodecyl ester.

8. The composition according to one of claims 1, 2, 3, 4, 5, 6 or 7, **characterized in that** component d) is selected from at least one mixture of d)i) at least one volatile C₈-C₁₆ isoparaffin and d)ii) at least one ester of linear or branched, saturated or unsaturated fatty alcohols having 2-30 carbon atoms with linear or branched, saturated or unsaturated fatty acids having 2-30 carbon atoms, which may be hydroxylated, and from at least one mixture of d)i) at least one volatile C₈-C₁₆ isoparaffin and d)ii) at least one benzoic acid ester of linear or branched C₈₋₂₂ alkanols.

9. The composition according to one of claims 1, 2, 3, 4, 5, 6, 7 or 8, **characterized in that** it contains triethyl citrate/ester/C₁₀-C₁₃ isoparaffin in ratios by weight of triethyl citrate/ester/C₁₀₋₁₃ isoparaffin of (1-1.3) : (0.6-1) : (1-3), preferably (1-1.3) : 1 : (1-1.5), also preferably (1-1.3) : (0.6-0.9) : (2.5-3), in particular 1 : 0.8 : 3.

10. The composition according to one of claims 1, 2, 3, 4, 5, 6, 7, 8 or 9, **characterized in that** it contains at least one organosiloxane-oxyalkylene copolymer which is selected from a linear polysiloxane-polyoxyethylene-polyoxypropylene block copolymer having the INCI name PEG/PPG-22/24 Dimethicone and a linear polysiloxane-polyoxyethylene-polyoxypropylene block copolymer having the INCI name PEG/PPG-10/2 Dimethicone, and mixtures thereof.

11. The composition according to claim 10, **characterized in that** it contains the at least one organosiloxane-oxyalkylene copolymer in a total quantity of from 0.01-5 wt. %, preferably from 0.1-3 wt.%, particularly preferably from 0.5-2 wt.%, most preferably from 0.7-1.5 wt.%, in each case based on the total weight of the propellant-free composition.

12. The composition according to one of claims 1, 2, 3, 4, 5, 6, 7, 8, 9, 10 or 11, **characterized in that** the oil mixture c) plus d) is selected from triethyl citrate/2-ethylhexyl palmitate/isodecane/isoundecane/isododecane/isotridecane, triethyl citrate/hexyldecyl laurate/isodecane/isoundecane/isododecane/isotridecane, triethyl citrate/2-ethylhexyl stearate/isodecane/isoundecane/isododecane/isotridecane, triethyl citrate/isopropyl myristate/isodecane/isoundecane/isododecane/isotridecane, triethyl citrate/isopropyl palmitate/isononane/isodecane/isoundecane/isododecane/isotridecane, triethyl citrate/2-ethylhexyl laurate/isodecane/isoundecane/isododecane/isotridecane, triethyl citrate/C₁₂-C₁₅ alkyl lactate/isodecane/isoundecane/isododecane/isotridecane, triethyl citrate/C₁₂-C₁₅ alkyl benzoate/isodecane/isoundecane/isododecane/isotridecane and triethyl citrate/di-C₁₂-C₁₃alkyl malate/isodecane/isoundecane/isododecane/isotridecane.

13. A non-therapeutic cosmetic method for reducing and/or regulating the formation of sweat and/or body odor, in which an effective quantity of a composition according to one of claims 1, 2, 3, 4, 5, 6, 7, 8, 9, 10, 11 or 12 is applied to the skin, preferably to the skin in the underarm region.

14. The non-therapeutic cosmetic use of an antiperspirant composition according to claim 1, 2, 3, 4, 5, 6, 7, 8, 9, 10, 11 or 12 for reducing and/or regulating sweating and/or body odor.

## Revendications

1. Composition anti-transpirante pour le soin du corps, confectionnée sous forme de suspension pulvérisable avec ou sans vecteur, contenant :
a) au moins une substance active anti-transpirante, les substances actives anti-transpirantes étant présentes en suspension insoluble,
b) 0 à 5 % en poids d'eau libre, rapporté au poids de la composition sans vecteur,
c) du citrate de triéthyle,
d) au moins une autre huile cosmétique liquide en conditions normales, comme support,
e) 0 à moins de 1 % en poids de cyclométhicone, rapporté au poids de la composition sans vecteur,
f) 0 à 3 % en poids d'éthanol, rapporté au poids de la composition sans vecteur,
**caractérisée en ce que** le rapport pondéral du citrate de triéthyle à la quantité totale d'huiles c) + d) + e) est de 40 à 50 % en poids.

2. Composition selon la revendication 1, **caractérisée en ce que** la quantité totale de citrate de triéthyle et d'au moins une autre huile cosmétique liquide en conditions normales d) + e) est de 30 à 95 % en poids, préférentiellement de 40 à 93 % en poids, particulièrement préférentiellement de 50 à 90 % en poids, extraordinairement préférentiellement de 53 à 63 % en poids, rapporté au poids total de la composition sans vecteur.

3. Composition selon la revendication 1 ou 2, **caractérisée en ce qu'**outre du citrate de triéthyle, il y est contenu au moins une huile liquide qui n'est pas une huile de silicone.

4. Composition selon la revendication 1, 2 ou 3, **caractérisée en ce que** l'huile support liquide en conditions normales d), qui n'est pas une huile de silicone, est choisie parmi les isoparaffines en C₈₋₁₆, en particulier l'isononane, l'isodécane, l'isoundécane, l'isododécane, l'isotridécane l'isotetradécane, l'isopentadécane et l'isohexadécane, ainsi que leurs mélanges, les mélanges d'isoparaffines en C₁₀₋₁₃ étant particulièrement préférentiels.

5. Composition selon la revendication 4, **caractérisée en ce que** l'au moins une isoparaffine en C₈₋₁₆ est contenue en une quantité totale de 25 à 50 % en poids, préférentiellement de 30 à 45 % en poids, particulièrement préférentiellement de 32 à 40 % en poids, extraordinairement préférentiellement de 33, 34, 35, 36,37, 38 ou 39 % en poids, rapporté au poids total de la composition sans vecteur.

6. Composition selon la revendication 1, 2, 3, 4 ou 5, **caractérisée en ce qu'**outre du citrate de triéthyle et l'au moins une isoparaffine en C₈₋₁₆, est contenue une huile cosmétique non liquide choisie parmi des huiles de silicone non-liquides et des huiles non de silicone non liquides.

7. Composition selon la revendication 1, 2, 3, 4, 5 ou 6, **caractérisée en ce que** l'au moins une huile non de silicone non liquide est choisie parmi des esters d'alcools gras linéaires ou ramifiés, saturés ou insaturés, avec de 2 à 30 atomes de carbone, avec des acides gras linéaires ou ramifiés, saturés ou insaturés, avec de 2 à 30 atomes de carbone, qui peuvent être hydroxylés, ainsi que parmi des esters de l'acide benzoïque d'alcools linéaires ou ramifiés en C₈₋₂₂, préférentiellement parmi le palmitate d'isopropyle, le myristate d'isopropyle, le stéarate d'isopropyle, le stéarate d'hexyldécyle, le laurate d'hexyldécyle, l'isononanoate d'isononyle, le palmitate de 2-éthylhexyle, le stéarate de 2-éthylhexyle, le stéarate d'isooctyle, le stéarate d'isononyle, le stéarate d'isocétyle, l'isononanoate d'isononyle, l'isononanoate d'isotridécyle, l'isononanoate de cétéaryle, le laurate de 2-éthylhexyle, l'isostéarate de 2-éthylhexyle, le cocoate de 2-éthylhexyle, le palmitate de 2-octyldodécyle, le 2-butyloctanoate de 2-butyloctyle, l'acétate de diisotridécyle, le laurate de n-hexyle, l'oléate de n-décyle, l'oléate d'oléyle, l'érucate d'oléyle, l'oléate d'érucyle, les lactates de C₁₂₋₁₅-alkyle, le malate de di-C₁₂₋₁₃-alkyle, le benzoate de C₁₂₋₁₅-alkyle, le benzoate d'isostéaryle, le benzoate d'éthylhexyle et le benzoate d'octyldodécyle.

8. Composition selon la revendication 1, 2, 3, 4, 5, 6 ou 7, **caractérisée en ce que** les composants d) sont choisis parmi au moins un mélange de d)i) au moins une isoparaffine liquide en C₈₋₁₆ et d)ii) au moins un ester d'alcool gras linéaire ou ramifié, saturé ou insaturé, ayant de 2 à 30 atomes de carbone, avec un acide gras linéaire ou ramifié, saturé ou insaturé, ayant de 2 à 30 atomes de carbone, pouvant être hydrolysé, ainsi que de d)i) au moins une isoparaffine liquide en C₈₋₁₆ et d)ii) au moins un benzoate d'alcools linéaires ou ramifiés en C₈₋₂₂.

9. Composition selon une des revendications 1, 2, 3, 4, 5, 6, 7 ou 8, **caractérisée en ce que** le mélange citrate de triéthyle / ester / isoparaffine en C₁₀₋₁₃ est contenu dans des rapports pondéraux citrate de triéthyle / ester / isoparaffine en C₁₀₋₁₃ de (1-1,3) : (0,6-1) : (1-3), préférentiellement (1-1,3) : (0,6-0,9) : (2,5-3), en particulier 1 : 0,8 : 3.

10. Composition selon une des revendications 1, 2, 3, 4, 5, 6, 7, 8 ou 9, **caractérisée en ce qu'**il y est contenu au moins un copolymère organosilane-oxyde d'alkyle, choisi parmi un copolymère bloc linéaire polysiloxane-polyoxyde d'éthylène-polyoxyde de propylène avec la désignation INCI PEG/PPG-22/24 diméthicone, et un copolymère bloc linéaire polysiloxane-polyoxyde d'éthylène-polyoxyde de propylène avec la désignation INCI PEG/PPG-10/2 diméthicone, ainsi que leurs mélanges.

11. Composition selon la revendication 10, **caractérisée en ce que** l'au moins un copolymère bloc linéaire polysiloxane-polyoxyde d'éthylène-polyoxyde de propylène est contenu dans une quantité totale de 0,01 à 5 % en poids, préférentiellement de 0,1 à 3 % en poids, particulièrement préférentiellement de 0,5 à 2 % en poids, extraordinairement préférentiellement de 0,7 à 1,5 % en poids, rapporté au poids total de la composition sans vecteur.

12. Composition selon une des revendications 1, 2, 3, 4, 5, 6, 7, 8, 9, 10 ou 11, **caractérisée en ce que** le mélange d'huiles c) + d) est choisi parmi citrate de triéthyle / palmitate de 2-éthylhexyle / isodécane / isoundécane / isododécane / isotridécane, citrate de triéthyle / laurate d'hexyldécyle / isodécane / isoundécane / isododécane / isotridécane, citrate de triéthyle / stéarate de 2-éthylhexyle / isodécane / isoundécane / isododécane / isotridécane, citrate de triéthyle / myristate d'isopropyle / isodécane / isoundécane / isododécane / isotridécane, citrate de triéthyle / palmitate d'isopropyle / isononane / isodécane / isoundécane / isododécane / isotridécane, citrate de triéthyle / laurate de 2-éthyhexyle / isodécane / isoundécane / isododécane / isotridécane, citrate de triéthyle / lactate de C₁₂₋₁₅-alkyle / isodécane / isoundécane / isododécane / isotridécane, citrate de triéthyle / benzoate C₁₂₋₁₅-alkyle / isodécane / isoundécane / isododécane / isotridécane et citrate de triéthyle / malate de di-C₁₋₁₃-alkyle / isodécane / isoundécane / isododécane / isotridécane,

13. Procédé cosmétique non thérapeutique de réduction et/ou de régulation de la formation de sueur et/ou d'odeurs corporelles, dans lequel une composition selon l'une des revendications 1, 2, 3, 4, 5, 6, 7, 8, 9, 10, 11 ou 12 est appliquée sur la peau dans une quantité efficace, préférentiellement sur la peau dans la région des aisselles.

14. Utilisation cosmétique non thérapeutique d'une composition anti-transpirante selon l'une des revendications 1, 2, 3, 4, 5, 6, 7, 8, 9, 10, 11 ou 12 pour réduire et/ou réguler la transpiration et/ou les odeurs corporelles.
